# EUROPÄISCHE PATENTSCHRIFT

(11) **EP 1 893 868 B1**
(45) Veröffentlichungstag und Bekanntmachung des Hinweises auf die Patenterteilung: **05.06.2013**
(21) Anmeldenummer: 06707449.2
(22) Anmeldetag: 07.03.2006
(51) Int. Cl.: F02P 17/12, G01N 33/00

(54) **SCHALTUNG ZUM ERFASSEN VERBRENNUNGSRELEVANTER GRÖSSEN**
CIRCUIT FOR DETECTING COMBUSTION-RELATED VARIABLES
CIRCUIT POUR SAISIR DES GRANDEURS CONCERNANT LA COMBUSTION

(30) Priorität: 13.06.2005 DE 102005027396; 28.06.2005 DE 102005030481; 14.09.2005 DE 102005044030
(43) Veröffentlichungstag der Anmeldung: 05.03.2008
(73) Patentinhaber: Stiebel Eltron GmbH & Co. KG, 37603 Holzminden (DE)
(72) Erfinder: NOLTE, Hubert, 37671 Höxter (DE); HERRS, Martin, 37671 Höxter (DE)
(74) Vertreter: Eisenführ, Speiser & Partner
(86) Internationale Anmeldenummer: PCT/EP2006/002077
(87) Internationale Veröffentlichungsnummer: WO 2006/133752

(56) Entgegenhaltungen:
- EP-A- 0 801 226
- WO-A-01/79803
- DE-C1- 19 916 205
- US-A- 4 601 193
- US-A- 5 755 206
- US-A- 5 769 049
- US-B1- 6 222 367
- ORRIN J E: "Controlling combustion with ionisation feedback" CONTROL, 1988. CONTROL 88., INTERNATIONAL CONFERENCE ON OXFORD, UK, LONDON, UK,IEE, UK, 1988, Seiten 65-69, XP006518796 ISBN: 0-85296-360-2

## Beschreibung

Die Erfindung betrifft eine Schaltung zum Erfassen verbrennungsrelevanter Größen in der Verbrennungsphase einer Verbrennungskraftmaschine, insbesondere eines Ottomotors mittels einer mit dem Zündimpuls beaufschlagten Zündelektrode und ein Verfahren zur Ermittlung verbrennungsrelevanter Größen eines Verbrennungsprozesses bei Verbrennungskraftmaschinen.

Derartige Verfahren sind in EP 0 801 226 A2 DE 196 49 278 A1 und US 4 601 193 beschrieben.

Mit Hilfe von Ionisationsmessungen im Brennraum von Verbrennungskraftmaschinen lassen sich detaillierte Aussagen über charakteristische Größen des Verbrennungsprozesses ableiten. Dies sind u. a. die Luftzahl der Verbrennung, der Brennbeginn, der Druckverlauf während der Verbrennung, die verschleppte Zündung, das Klopfen, Zündaussetzer o. dgl.

Hierzu wird ein Sensorelement üblicherweise an einer repräsentativen Position im Brennraum angeordnet. Typischerweise handelt es sich hierbei um ein elektrisch leitfähiges Element, welches mit Hilfe eines Isolators gegenüber dem Potential des Brennraums isoliert ist.

Über eine Signalleitung wird eine Messspannung angelegt, und in Abhängigkeit des Verbrennungsverlaufes fließt ein Strom durch die ionisierten Verbrennungsgase vom Sensorelement zur Brennraummasse. Ein derartiger Strom wird nachfolgend ausgewertet.

lonisationsmessvorrichtungen werden typischerweise in den Zündspannungskreis eines Verbrennungsmotors geschaltet. Der Zündspannungskreis besteht hierbei aus einer Zündspuleneinheit, einer Zündkerze sowie einer elektrischen Verbindung zwischen beiden. Die Zündspuleneinheit kann vorteilhafterweise die Leistungselektronik zum Anschalten der Primärspule an die Energieversorgung zum Auslösen des Zündfunkens enthalten.

Dabei wird die Energie zum Durchführen einer Ionisationsmessung entweder bei der Zündung dem Zündkreis entnommen oder wird von einer externen Energiequelle zugeführt. Ein Ionisationsstrom bewirkt einen Spannungsabfall an einem Messwiderstand und wird über eine Messleitung an die den Ionisationsverlauf weiterverarbeitenden und auswertenden Einheiten des Motormanagements weitergeleitet. Ein derartiger Aufbau weist jedoch den Nachteil auf, dass eine zusätzliche Leitung erforderlich ist.

Die Auswertung von Ionisationssignalverläufen bei diskontinuierlichen Verbrennungen bei Verbrennungskraftmaschinen (Otto, Diesel, etc.) zur Ermittlung von Motorparametern wie Luftzahl, verschleppte Verbrennung, Zündaussetzer, Klopfen, opt. Zündzeitpunkt erfolgt bislang über Funktionsanalyse des lonenstromverlaufs hinsichtlich 'geometrischer' Daten wie Maximum, Lage des Maximums, Integral, Flächenschwerpunkt, Steigung, Maximum der Steigung etc.

Systematische Störeinflüsse überlagern die Auswertung der geometrischen Daten, wie beispielsweise Zündkerzenabbrand, Zündkerzenverschmutzung, unterschiedliche Kraftstoffqualität und damit Leitfähigkeit der Flamme, stochastische Schwankungen der Signalhöhe durch unterschiedliche Ausbreitung der Flammenfront im Brennraum, Einflüsse aus der Zündrestspannung (auch Abhängig der Aufladung des Motors), Einflüsse anderer als der zu ermittelnde Motorparameter.

Mit Hilfe von Kalibrier- und Wichtungsfunktionen lässt sich die Auswertung hinsichtlich des gewünschten Parameters nur bedingt verbessern.

Es ist Aufgabe der Erfindung, die Genauigkeit der Erfassung des Ionisationssignals zu verbessern.

Diese Aufgabe wird durch eine Schaltung gemäß Anspruch 1 und durch ein Verfahren gemäß Anspruch 13 gelöst.

Hierbei ist der lonisationsmesskreis an die Zündelektrode angeschaltet und über Entkopplungsmittel von der Zündspule entkoppelt. In der Zündphase wird mit einem Zündstrom ein Spannungspotential in einer Messspannungs-Speichereinheit aufgebaut und das Spannungspotential liegt in wenigstens einer Messphase über einem Messwiderstand an einer Zündelektrode an, wobei die Zündspannung bei Unterschreiten einer Schwellspannung von der Messspannungs-Speichereinheit oder dem Messwiderstand entkoppelt ist.

Gemäß einem Aspekt der vorliegenden Erfindung ist der lonisationsmesskreis zwischen dem Zündspannung führenden Anschluss der Sekundärseite des Zündtransformators und der Zündelektrode geschaltet und zwischen dem Bezugspotential und zwischen der dem Bezugspotential zugeordneten Anschluss der Sekundärseite des Zündtransformators ist ein elektronischer Schalter geschaltet, der durch eine Steuereinheit während des Zündimpulses zur Weiterleitung des Zündsignals leitend geschaltet wird und zumindest während der lonisationsmessphase sperrend geschaltet ist. Ein Messspannungsspeicherkreis ist zwischen dem die Zündspannung führenden Anschluss der Sekundärseite des Zündtransformators und der Zündelektrode geschaltet, und ein elektronischer Schalter ist zwischen einem Bezugspotential und einem dem Bezugspotential zugeordneten Anschluss der Sekundärseite des Zündtransformators geschaltet. Die Schaltung kann in einen Zündtransformator integriert sein. Die Schaltung weist einen lonisationsmesskreis auf, welcher an die Zündelektrode angeschaltet ist und über Entkopplungsmittel vom Zündtrafo entkoppelt ist. Es wird ein Verfahren vorgeschlagen, bei welchem in der Zündphase mit einem Zündstrom ein Spannungspotential in einer Messspannungs-Speichereinheit aufgebaut wird, wobei das Spannungspotential in wenigstens einer Messphase über einem Messwiderstand an einer Zündelektrode anliegt, wobei die Zündspannung bei Unterschreiten einer Schwellspannung von der Messspannungs-Speichereinheit oder dem Messwiderstand entkoppelt ist.

Mit einem elektronischen Schalter, der während des Zündimpulses zur Weiterleitung des Zündsignals leitend geschaltet wird und im Anschluss an den Zündimpuls zur Ionisationsmessung sperrend geschaltet wird, wird die Zündrestspannung vom lonisationsmesskreis entkoppelt. Dieser Schalter wird von einer Zündsteuerungseinheit angesteuert, die den Ablauf der Zündung steuert.

In vorteilhafter Weise lässt sich das Verfahren auch im Zusammenspiel mit einer Zündfunkensteuerung betreiben. Die Zündspannungssteuereinheit steuert dabei die Abschaltung des Zündimpulses nach vorbestimmter Dauer, indem die Primärwicklung des Zündtransformators durch einen Kurzschluss nierderimpedant belastet wird. Die sich beim Abbau der Restenergie des Zündtrafos einstellende Spannung auf der Sekundärseite des Zündtransformators wird durch den gesperrten elektronischen Schalter nicht an den lonisationsmesskreis weitergeleitet.

Gemäß einem weiteren Aspekt der vorliegenden Erfindung wird eine definierte Zündenergie durch eine vorgegebene erste Brenndauer eines Zündfunkens vorgegeben. Nach dem Brennende des ersten Zündfunkens erfolgt eine Messung einer Ionisation im Brennraum der Verbrennungskraftmaschine zur Ermittlung von Ionisationsmesswerten. Diese werden mit vorgegebenen Werten verglichen und im Falle einer zu geringen Ionisation, die einem noch nicht entzündeten Brennstoff-Luftgemisch entspricht, eine zweite Zündspannung an die Zündkerze gelegt. Nach dem Brennende des zweiten Zündfunkens wird erneut die Ionisation gemessen und durch Vergleich ermittelt, ob eine Zündung im Brennraum erfolgt ist. Nach Brennende eines Zündfunkens wird somit überwacht, ob schon eine Zündung erfolgt ist und das Gemisch brennt. Der Zyklus des Erzeugens eines Zündfunkens und der Ionisationsmessung mit Vergleich erfolgt so lange, bis ein Ionisationswert gemessen wird, der einem entzündeten Gemisch entspricht.

Mittels einer getakteten Zündung kann die dem Brennraum zugeführte Zündenergie in definierten Intervallen zugeführt werden. In vorteilhafter Weise ist die Zündenergie zeitgesteuert, d.h. der Zündfunke brennt für einen vorgegebenen Zeitraum und wird dann unterbrochen. Nach dem Ende des Brennens des Zündfunkens wird sofort mittels einer Ionisations-Messschaltung gemessen, ob das Gemisch entflammt ist. Hierzu werden Ionisationsmesswerte mit lonisations-Sollwerten verglichen, und abhängig von der Differenz wird entschieden und erkannt, ob eine Verbrennung stattfindet. Findet eine Verbrennung statt, wird die Ionisation in vorteilhafter Weise weiter gemessen, um das Brennen des Gemisches weiter zu verfolgen und weitere verbrennungsrelevante Parameter, wie Klopfen und Lambda-Wert zu ermitteln. Bleibt die Entflammung des Gemisches aus oder findet sie nicht vollständig statt, wird erneut gezündet und dem Brennraum wiederum eine definierte Zündenergie zugeführt, was in vorteilhafter Weise zeitgesteuert erfolgt, so dass der Zündfunke für einen vorgegebenen Zeitraum erneut brennt. Hiernach erfolgt wiederum eine Ionisationsmessung, um zu ermitteln, ob das Gemisch brennt oder nicht. Der Zyklus des Zündens mit einer abwechselnden Ionisationsmessung erfolgt so lange, bis das Gemisch entzündet ist oder der Verbrennungszyklus beendet ist. Hierbei dient die Zündkerze als Zündelektrode sowie als Messsensor.

In einer vorteilhaften Ausgestaltung der Erfindung wird kein weiterer Zündfunke nach einer erfolgten sicheren Verbrennung erzeugt und die Ionisation im Brennraum weiter gemessen, um weitere Motorparameter, wie Klopfen und Lambda-Wert, zu erfassen. Sollte die Ionisation einen unzulässigen Verlauf aufweisen und zusammenbrechen oder sollten beim Vergleich mit vorgegebenen Werten Abweichungen auftreten, was auf ein vorzeitiges Verlöschen der Flamme deutet, wird wieder eine Zündspannung angelegt, um das Gemisch nochmals zu zünden. Hiermit wird zur Zündung des Gemisches die erforderliche Energie nach Bedarf bereitgestellt und mittels Ionisationssignal überprüft, ob eine Zündung erfolgt ist. In einer vorteilhaften Ausführungsform wird die Brenndauer als Parameter für die Zündenergie gesteuert und die Brenndauer minimiert. In vorteilhafter Weise wird dazu ermittelt, wie lange der erste Zündfunke brennen muss, um unter verschiedenen Betriebsbedingungen eines Motors eine sichere Zündung zu erreichen. Dies erfolgt mit selbst adaptiven Verfahren und in vorteilhafter Weise in einem Mikroprozessor.

Weiterhin ist durch das Verfahren und die Einrichtung eine Zündspule mit einem geringeren Arbeitsspeichervermögen verwendbar, da der erste Zündfunke nicht für alle Zündungen über den gesamten Arbeitsbereich der Maschine ausgelegt ist. Weiterhin ist es vorteilhaft, den Zündfunken sofort nach erfolgter Zündung zu beenden, um mittels der folgenden Ionisationsmessung schnell Signale über den Brennverlauf, wie Lambda-Wert, Klopfen und verschleppte Verbrennung, zu erhalten. Nach dem letzten Zündimpuls wird das Ionisationssignal hierzu weiter gemessen.

Gemäß einem weiteren Aspekt der Erfindung wird für die Weiterleitung des lonisationssignals an die Auswerteeinheit die selbe Leitung benutzt, die das Signal zur Triggerung der Leistungselektronik zum Auslösung des Zündimpulses führt. Das Ionisationssignal wird in der Zündeinheit über entsprechende Mittel mit dem Auslösesignal für die Zündung zusammengeschaltet, so können beide Signale. über eine Leitung weitergeleitet werden. In der den Zündimpuls steuernden und das Ionisationssignal weiterverarbeitenden Einheit werden die Signale wieder über entsprechende Mittel getrennt und getrennt voneinander verarbeitet.

Die Erfindung betrifft den Gedanken, die Ähnlichkeit des aktuell gemessenen Ionisationssignalverlaufes mit verschiedenen für bestimmte Betriebspunkte bzw. Motorparameter charakteristische gespeicherte Ionisationssignalverläufe zu bestimmen. Aus dem Verhältnis der ermittelten Ähnlichkeitsgrade wird dann der Wert des Motorparameters berechnet. Somit wird bei dem Verfahren nicht nur eine oder wenige geometrische Größe(n) des Ionisationssignalverlaufes ermittelt, sondern der gesamte Signalverlauf bewertet.

Weitere Aspekte der Erfindung sind Gegenstand der Unteransprüche.

Nachfolgend werden die Ausführungsbeispiele und Vorteile der vorliegenden Erfindung unter Bezugnahme auf die beigefügten Zeichnungen näher erläutert:
- Fig. 1: zeigt ein schematisches Schaltbild einer Motorsteuerung gemäß einem ersten Ausführungsbeispiel,
- Fig. 2: zeigt ein schematisches Schaltbild einer Schaltung zur Zündung und Ionisationsmessung gemäß einem zweiten Ausführungsbeispiel,
- Fig. 3: zeigt ein schematisches Schaltbild einer Schaltung zur Zündung und Ionisationsmessung gemäß einem dritten Ausführungsbeispiel,
- Fig. 4: zeigt ein schematisches Schaltbild einer Schaltung zum Zünden von Verbrennungen gemäß einem dritten Ausführungsbeispiel,
- Fig. 5: zeigt ein Schaltbild einer Schaltung zum Zünden in einer Verbrennungskraftmaschine gemäß einem vierten Ausführungsbeispiel,
- Fig. 6: zeigt ein Schaltbild einer Motorelektronikeinheit gemäß einem fünften Ausführungsbeispiel,
- Fig. 7: zeigt ein Schaltbild eines Kopplungsmittels von Fig. 5 gemäß einem siebten Ausführungsbeispiel,
- Fig. 8: zeigt ein Schaltbild einer Zündeinheit gemäß einem achten Ausführungsbeispiel,
- Fig. 9: zeigt eine schematische Darstellung eines Zündkerzensteckers gemäß einem neunten Ausführungsbeispiel,
- Fig. 10: zeigt eine schematische Darstellung mehrerer Zündkerzen und eines Zündverteilers gemäß einem zehnten Ausführungsbeispiel,
- Fig. 11: zeigt eine schematische Darstellung mehrerer Zündkerzen und eines Zündverteilers gemäß einem elften Ausführungsbeispiel
- Fig. 12: zeigt eine schematische Darstellung von mehreren Zündkerzen sowie eines Zündverteilers gemäß einem zwölften Ausführungsbeispiel,
- Fig. 13: zeigt eine schematische Darstellung eines Aufbaus eines Zündkerzensteckers gemäß einem dreizehnten Ausführungsbeispiel,
- Fig. 14: zeigt eine schematische Darstellung einer Schaltung zum Auswerten von Ionisationssignalverläufen gemäß einem vierzehnten Ausführungsbeispiel,
- Fig. 15a: zeigt die relative Signalamplitude von Referenzkurven sowie der gemessenen Ionisationskurve IO,
- Fig. 15b: zeigt die relativen Anteile der gemessenen Ionisationskurve IO zu den jeweiligen Referenzsignalverläufen,
- Fig. 16a und 16b: zeigen einen Graph von Referenzsignalverläufen und einem lonisationssignal sowie einen Graph einer Zuordnung der Parameter,und
- Fig. 17a und 17b: zeigen einen Graph von Referenzkurven sowie eines gemessenen Ionisationssignals sowie eine Zuordnung der Parameter, und

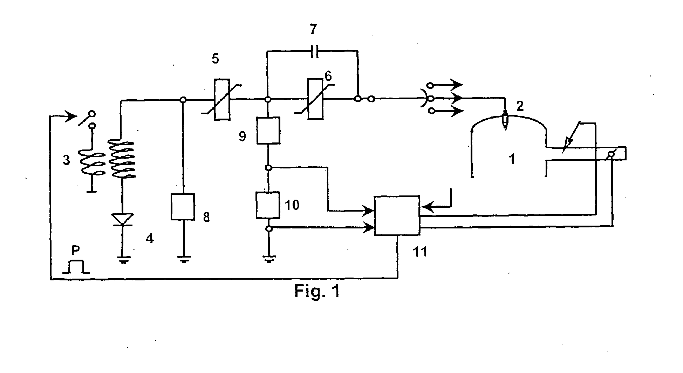

Fig. 1 zeigt ein Schaltbild einer Schaltung zum Zünden und zur Ionisationsmessung gemäß einem ersten Ausführungsbeispiel. In einem Brennraum 1 einer Verbrennungskraftmaschine ist mindestens eine Zündkerze 2 angeordnet.

Ein lonisationsmesskreis ist durch Entkopplungsmittel 5,8 von der Zündspule 3 entkoppelt. Der während des Funkenüberschlages durch die Zündkerze fließende Zündstrom passiert die Entkopplungsmittel 5, 8 und lädt den Messspannungs-Speicher 6,7 im Ionisationsmesskreis auf. Nach der Beendigung des Funkenüberschlages entkoppeln die Entkopplungsmittel 5,8 den lonisationsmesskreis, bestehend aus dem Kondensator 7, dem Varistor 6, einem Vorwiderstand 9 und einem Messwiderstand 10, von der Zündrestspannung.

Der lonisationsmesskreis weist dazu mindestens eine Messspannungs-Speichereinheit 6,7, einen Ionisationsmesswiderstand 9, 10 und die im Brennraum 1 angeordnete Zündkerze 2, welche als Ionisationssensor verwendet wird, auf.

Die Messspannungs-Speichereinheit 6,7 besteht in vorteilhafter Weise aus Parallelschaltung eines elektrischen Energiespeichers, insbesondere Kondensators 7, und eines spannungsbegrenzenden Bauteils, insbesondere eines Varistors 6 oder einer Zenerdiode.

In der Phase, in der der Zündstrom fließt, wird die Messspannungs-Speichereinheit 6,7 über den Zündstrom auf- bzw. nachgeladen, die Ladespannung wird mit Hilfe des spannungsbegrenzenden Bauteils 6 auf dessen Schwellwert stabilisiert.

In der nachfolgenden Verbrennungsphase liegt die aufgeladene Messspannungs-Speichereinheit 6,7 über einer Messwiderstandskombination 9,10 an der Zündelektrode 2 an. Ein dem Ionisationsstrom proportionaler Spannungsabfall kann als Ionisationssignal am Messwiderstand 10 abgegriffen und weiterverarbeitet werden.

Eine vorteilhafte Ausgestaltung der Messwiderstandskombination 9,10 ist die Reihenschaltung von Widerständen 9, 10 zur Erhöhung der Gesamtimpedanz, wobei das Ionisationssignal in vorteilhafter Weise über dem an Schaltungsmasse angeschalteten Widerstand 10 abgegriffen wird.

Dieses Ionisationssignal wird im folgenden in der Auswerteeinheit 11 weiterverarbeitet. Hier kann es auch hinsichtlich verbrennungsrelevanter Größen ausgewertet werden.

Ein Entkopplungsmittel entkoppelt den Einfluss der Zündrestspannung vom Ionisationsmesskreis, in dem in vorteilhafter Weise der lonisationsmesskreis über ein spannungsbegrenzendes Bauteil 5 von der Zündspule getrennt ist. Des weiteren können auch Bauteile parallel zur Sekundär- oder Primärseite der Zündspule geschaltet werden, die ein Abklingen der Zündrestspannung beschleunigen. Dies geschieht in vorteilhafterweise durch den Widerstand 8.

Die in Reihe mit der Sekundärseite der Zündspule geschaltete Diode 4 sperrt ein positives Potential am Zündspulenausgang, welches sich ohne die Diode schon beim Anlegen der Primärspannung an die Primärwicklung der Zündspule 3 einstellen würde, was aber unerwünscht ist.

Das erste Ausführungsbeispiel betrifft eine Schaltung zum Erfassen verbrennungsrelevanter Größen in einer Verbrennungsphase einer Verbrennungskraftmaschine. Die Verbrennungsphase wird durch eine mit einem Zündimpuls beaufschlagte Zündelektrode ausgelöst. Die Schaltung weist einen lonisationsmesskreis mit mindestens einem Messwiderstand 8, 9 auf. Der lonisationsmesskreis weist eine Messspannungs-Speichereinheit 6, 7 auf. Die Speichereinheit ist mit der Zündelektrode 2 gekoppelt und ist über ein Entkopplungsmittel von der Zündspule der Verbrennungskraftmaschine entkoppelt.

Die Messspannungs-Speichereinheit weist eine elektrische Energie speichernde Komponente (ein Kondensator) und eine spannungsbegrenzende Komponente (ein Varistor, eine Zenerdiode, eine Funkenstrecke oder ein Gasableiter) auf.

Das erste Ausführungsbeispiel betrifft ebenfalls ein Verfahren zur Erfassung verbrennungsrelevanter Größen in einem Verbrennungsprozess einer Verbrennungskraftmaschine. Die Verbrennungsvorgänge werden durch einen Zündimpuls initiiert. Die verbrennungsrelevanten Größen werden in Abhängigkeit eines durch die Flamme beeinflussten Ionisationssignals ermittelt. Ein Spannungspotenzial wird in einer Messspannungs-Speichereinheit in der Zündphase durch einen Zündstrom aufgebaut. Das Spannungspotenzial wird in mindestens einer Messphase über einen Messwiderstand an einer Zündelektrode angelegt, wobei die Zündspannung bei Unterschreiten einer Schwellspannung die Messspannungs-Speichereinheit oder den Messwiderstand von der Messspannung entkoppelt.

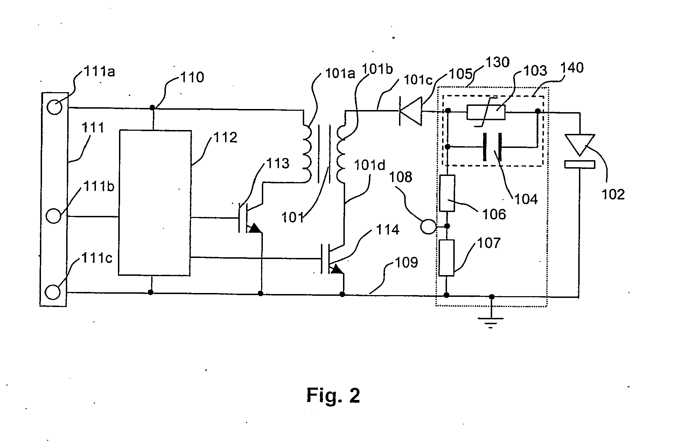

Fig. 2 zeigt ein schematisches Schaltbild einer Schaltung zur Zündung und lonisationsmessung gemäß einem zweiten Ausführungsbeispiel. In Fig. 2 ist ein Anschluss 111 gezeigt, der die Schaltung zur Zündung und Ionisationsmessung mit der Motorsteuerelektronik verbindet. Der Anschluss 111a ist mit der positiven Versorgungsspannung, Anschluss 111c mit dem Massepotential und Anschluss 111 b mit der Steuerleitung für die Zündimpulsauslösung verbunden. Die Zündsteuereinheit 112 ist an den Anschluss 111 angeschaltet und formt das Signal für die Ansteuerung des Leistungshalbleiters 113, der die Primärwicklung 101a des Zündtransformators 101 über die Pfade 110 und 109 an die Versorgungsspannung schaltet. Wenn die Zündsteuereinheit 112 den Leistungshalbleiter 113 wieder ausschaltet, wird auf der Sekundärwicklung 101 b des Zündtransformators 101 die Zündspannung induziert.

Zumindest während des Zündimpulses schaltet die Zündsteuereinheit 112 den durch einen Halbleiter gebildeten elektronischen Schalter 114 leitend, so dass der Zündstrom diesen passieren kann. Der Zündstrom passiert das Entkopplungsbauteil 105, welches als Diode ausgeführt ist, in Durchlassrichtung und lädt den Messspannungsspeicherkreis 140 auf, der durch ein spannungsstabilisierendes Bauteil 103 parallel zu einem Kondensator 104 gebildet ist. Der Messspannungs-Speicherkreis 140 entspricht der Messspannungs-Speichereinheit 6, 7 gemäß dem ersten Ausführungsbeispiel. Der Zündstrom passiert des weiteren die Zündelektrode 102, die Bestandteil einer in einem Verbrennungsraum einer Verbrennungskraftmaschine angeordneten Zündkerze ist und fließt über den Massepfad 109 und den elektronischen Schalter 114 zur Sekundärwicklung des Zündtransformators zurück. Nach Beendigung des Funkenüberschlags an der Zündelektrode 102 und Abbrechen des Zündstroms schaltet die Zündsteuereinheit 112 den durch einen Halbleiter gebildeten elektronischen Schalter 114 sperrend, so dass der Einfluss der noch im Zündtransformator 101 verbleibenden Restenergie auf die nun folgende Ionisationsmessung unterdrückt wird und somit die Zündrestspannung vom lonisationsmesskreis 130 abgekoppelt ist.

Die Ionisationsmessung vollzieht erfolgt ich im lonisationsmesskreis 130, in dem der durch den Zündimpuls aufgeladene Messspannungsspeicherkreis 140 die Messspannung über die Ionisationsmesswiderstände 106 und 107 an die Zündelektrode 102 anlegt.

In der Verbrennungsphase wird das Plasma im Brennraum zwischen der Zündelektrode 102 und der Brennraummasse elektrisch leitfähig, es fließt ein lonisationsstrom, der als Spannungsabfall über die Widerstände 106 und 107 gemessen werden kann. An Anschluss 108 ist während der lonisationsmessphase eine dem Ionisationsstrom proportionale Spannung abgreifbar, während des Zündvorganges ist an Anschluss 108 eine der Zündspannung proportionale Spannung abgreifbar.

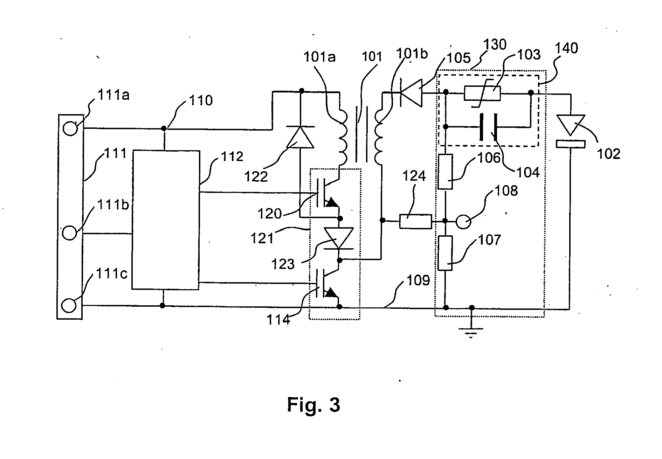

Fig. 3 zeigt ein schematisches Schaltbild einer Schaltung zur Zündung und lonisationsmessung gemäß einem dritten Ausführungsbeispiel. Fig. 3 zeigt insbesondere ein Schaltbild mit einer alternativeren Funkensteuerung. Mit Hilfe einer Funkensteuerung kann die im Zündtransformator 101 befindliche Restenergie während der Zündung durch eine an den Zündtransformator 101 angeschaltete Senke derart abgeleitet werden, dass der Zündfunke erlischt. Damit wird dem Brennraum 161 zur Zündung des Gemisches eine definierte Zündenergie bei einer definierten Brenndauer des Zündfunkens zugeführt. Als vorteilhafte Ausführung sind zwei Leistungshalbleiter 120 und 114 im Primärstrompfad 121 des Zündtransformators 101 verschaltet.

Zur Einbringung von Energie in den Zündtransformator 101 werden durch die Zündsteuereinheit beide Leistungshalbleiter 120 und 114 leitend geschaltet, der Primärstrom passiert dabei auch die sich im Primärstrompfad 121 befindliche Diode 123. Zur Erzeugung der Zündspannung wird im Anschluss daran der Leistungshalbleiter 120 sperrend geschaltet, die Sekundärwicklung des Zündtransformators wird durch den leitend geschalteten Leistungshalbleiter 114 an Bezugspotential gelegt, und die induzierte Zündspannung wird über die lonisationsmessschaltung 130 zur Zündelektrode 102 geleitet. Zur Beendigung des Zündfunkenbrennens wird durch die Zündsteuereinheit 112 der Leistungshalbleiter 114 gesperrt und der Leistungshalbleiter 120 leitend geschaltet.

Die Diode 122 und der Leistungshalbleiter 120 bilden einen Kurzschluss für die Restenergie des Zündtransformators 101. Der gesperrte Leistungshalbleiter 114 entkoppelt dann den Zündtransformator 101 von der Ionisationsmessschaltung 130 und die folgende Ionisationsmessung wird sehr exakt.

Der Widerstand 124 kompensiert eine Signalverfälschung durch parasitäre Kapazitäten parallel zur Schaltstrecke des Leistungshalbleiters 114.

In einer vorteilhaften Ausgestaltung bei der Ansteuerung der Leistungshalbleiter 114 und 120 durch die Zündsteuerung 112 erfolgt in der Funkenausschaltphase die Sperrung des Leistungshalbleiters 114 eine kurze Zeitspanne vor Öffnung des Leistungshalbleiters 120; die parasitäre Kapazität des Leistungshalbleiters 114 wird dabei vorgeladen.

Das zweite und dritte Ausführungsbeispiel betrifft eine Schaltung zur Erfassung verbrennungsrelevanter Größen einer Verbrennungskraftmaschine. Die Schaltung weist eine lonisationsmesseinrichtung mit mindestens einem lonisationsmesswiderstand auf. Ein Messspeicherkreis ist zwischen einem ersten Anschluss der Sekundärseite des Zündtransformators, welcher die Zündspannung führt, und der Zündelektrode gekoppelt. Ein elektronischer Schalter ist zwischen einem Bezugspotenzial und einem dem Bezugspotenzial zugeordneten zweiten Anschluss der Sekundärseite des Zündtransformators gekoppelt.

Eine weitere Entkopplungseinrichtung kann zwischen dem ersten Anschluss der Sekundärseite des Zündtransformators und der Ionisationsmessschaltung gekoppelt werden. Die Entkoppeleinrichtung kann eine Diode, eine Zenerdiode und/oder einen Varistor aufweisen.

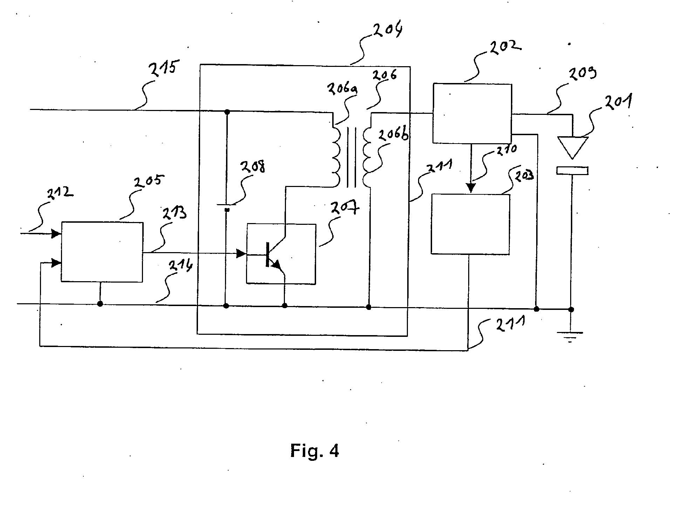

Fig. 4 zeigt ein Blockschaltbild der Einrichtung zum Zünden von Verbrennungen gemäß einem vierten Ausführungsbeispiel. Zum Auslösen einer Zündung wird über eine Signalleitung 212 zur Auslösung einer Zündung eine Zündauslöseanforderung an ein Mittel 205 zur Steuerung des Zündspannungsverlaufes gekoppelt. Über die Signalleitung 213 wird vom Mittel 205 zur Steuerung des Zündspannungsverlaufes ein Steuersignal zum Schalten eines Halbleiterschalters 207 im Mittel 204 zum Erzeugen der Zündspannung gegeben. Beim Durchschalten des Halbleiterschalters 207 wird an eine Primärwicklung 206a eines Zündtransformators 206 eine Zündspannungsversorgung 208 angeschaltet. Sperrt der Halbleiterschalter 207 - gesteuert durch das Steuersignal - wieder, wird in einer Sekundärwicklung 206b des Zündtrafos 206 die Zündspannung induziert. Die Zündspannung passiert einen Ionisationsdetektor 202 und gelangt über eine Verbindung 209 zur Zündkerze 201, wo ein Funkenüberschlag zum Zünden des Gemisches im Brennraum entsteht. Nach Beendigung des Funkenüberschlages misst der Ionisationsdetektor 202 die durch Flammenbildung erfolgende Ionisation im Brennraum. Das Messsignal gelangt über eine Leitung 210 zur Auswerteeinheit 203 für Ionisationsmesswerte. Hier wird bewertet, ob eine Entflammung des Gemisches im Brennraum stattgefunden hat. Bewertungsgrundlage ist der Vergleich des gemessenen Ionisationssignals nach Beendung des Funkenüberschlages an der Zündkerze 201 mit Ionisationssollwerten. Das Ergebnis der Bewertung wird über eine Leitung 211 an die Mittel 205 zur Steuerung des Zündspannungsverlaufes weitergeleitet. Hat keine Entflammung stattgefunden, wird durch das Mittel 205 zur Steuerung des Zündspannungsverlaufes eine weitere Zündung im selben Arbeitstakt ausgelöst. Hat eine Entflammung stattgefunden, wird durch das Mittel 205 zur Steuerung des Zündspannungsverlaufes keine weitere Zündung im selben Arbeitstakt ausgelöst.

Das vierte Ausführungsbeispiel bezieht sich auf eine Einrichtung zum Zünden von Verbrennungen in Verbrennungskraftmaschinen. Es wird eine Zündkerze zum Zünden der Verbrennung vorgesehen, wobei die Zündkerze als Sensor für eine durch die Verbrennung hervorgerufene Ionisation verwendet wird. Eine Ionisationsmessschaltung wird zum Messen einer Ionisation im Brennraum vorgesehen, und eine Auswerteeinheit für die Ionisationswerte sowie Mittel. zum Erzeugen der Zündspannung werden vorgesehen. Ein Mittel zur Steuerung des Zündspannungsverlaufes wird an die Ionisationsauswerteeinheit angeschaltet, und der Zündspannungsverlauf wird in Abhängigkeit der Ionisationswerte geregelt.

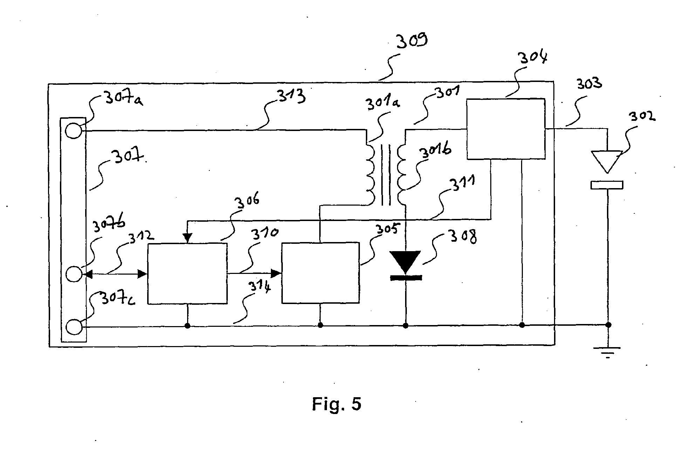

Fig. 5 zeigt ein Blockschaltbild einer Vorrichtung für die Zündung und die Generierung des Ionisationssignals gemäß einem fünften Ausführungsbeispiel. Diese Vorrichtung weist eine Zündspule 301, einen Ionisationsdetektor 304, eine Leistungselektronik-Zündungseinheit 305, ein Kopplungsmittel 306 sowie eine Diode 308 auf. Die Vorrichtung ist ferner über eine Verbindungsleitung 303 mit einer Zündkerze 302 verbunden. Die Vorrichtung weist ferner einen Anschlussstecker 307 mit drei Polen 307a, 307b und 307c auf.

Die Zündspule 301 besteht aus Primärwicklung 301 a und Sekundärwicklung 301 b. Die Primärwicklung 301 a ist an ihrem einen Anschluss an die Spannungsversorgungsleitung 313 geschaltet, die üblicherweise das Potential des Pluspols des KFZ-Bordnetzes führen kann. Die Primärwicklung 301a ist an ihrem zweiten Anschluss an die Leistungselektronik-Zündeinheit 305 angeschaltet. Die Leistungselektronik-Zündeinheit 305 weist einen Halbleiterschalter auf, der bei einem Triggerimpuls auf der Zündauslöseleitung 310 den zweiten Anschluss der Primärwicklung 301 a gegen Spannungsversorgungsmasse 314 schaltet, die üblicherweise das Potential des Minuspols des KFZ-Bordnetzes führt.

Die Zündspule 301 wirkt als Trafo, d. h. in der Sekundärwicklung 301 b induziert sich die Zündspannung, die zum Ionisationsdetektor 304 weitergeleitet wird. Die am zweiten Anschluss der Sekundärwicklung 301 b angeschaltete Diode 308 bewirkt eine Sperrung eines potenzialmäßig unerwünschten Anteils der Zündspannung.

Der Ionisationsdetektor 304 leitet den Zündimpuls über Verbindungsleitung 303 an die Zündkerze 302. Der Ionisationsdetektor 304 gemäß dem fünften Ausführungsbeispiel entspricht im Wesentlichen dem lonisationsmesskreis gemäß dem ersten, zweiten oder dritten Ausführungsbeispiel. Der Rückfluss des Zündstroms erfolgt über die Spannungsversorgungsmasse 314. Nach Abreißen des Zündstroms legt der Ionisationsdetektor 304 eine Messspannung an die Zündkerze 302 an. In Abhängigkeit des Verbrennungsablaufs an der Zündkerze 302 fließt ein Ionisationsstrom von der Zündelektrode der Zündkerze 302 zur Brennraummasse. Im Ionisationsdetektor 304 wird proportional zum Ionisationsstrom eine lonisationsmessspannung generiert, die an die lonisationsmessleitung 311 angelegt wird. An das Kopplungsmittel 306 ist sowohl die lonisationsmessleitung 311 als auch die Zündauslöseleitung 310 angeschaltet. Beide Signale werden im Kopplungsmittel 306 auf der Zünd/lonisationsleitung 312 zusammengeschaltet. Dieses Kopplungsmittel 306 koppelt die Ionisationsmessleitung 311 und die Zündauslöseleitung 310 in selektiver Weise an die Zünd/lonisationsleitung 312 an. Die Zünd/lonisationsleitung 312 stellt die Signalleitung dar, mit der das Motormanagement einer Verbrennungskraftmaschine sowohl den Zündimpuls auslöst als auch das Ionisationssignal zugeführt bekommt.

Sendet das Motormanagement einen Triggerimpuls für die Zündauslösung, so wird dieser vom Koppelmittel 306 über Zündauslöseleitung 310 an die Leistungselektronik 305 weitergeleitet. Eine unerwünschte Signalveränderung durch die Anschaltung des Ionisationsdetektor 304 über die Ionisationsrnessleitung 311 darf nicht erfolgen. Wenn der Ionisationsdetektor 304 ein Ionisationsstrom detektiert und die lonisationsmessspannung über die lonisationsmessleitung 311 an das Kopplungsmittel 306 weiterleitet, so muss das Kopplungsmittel 306 diese Spannung an die Zünd/Ionisationsleitung 312 weiterleiten. Eine unerwünschte Signalveränderung durch die Anschaltung der Leistungselektronik 305 über Zündauslöseleitung 310 darf nicht erfolgen.

Spannungsversorgungsleitung 313, Spannungsversorgungsmasse 314 und Zünd/lonisationsleitung 312 werden auf des Anschlussstecker 307 geschaltet und belegen die Pole 307a, 307b und 307c.

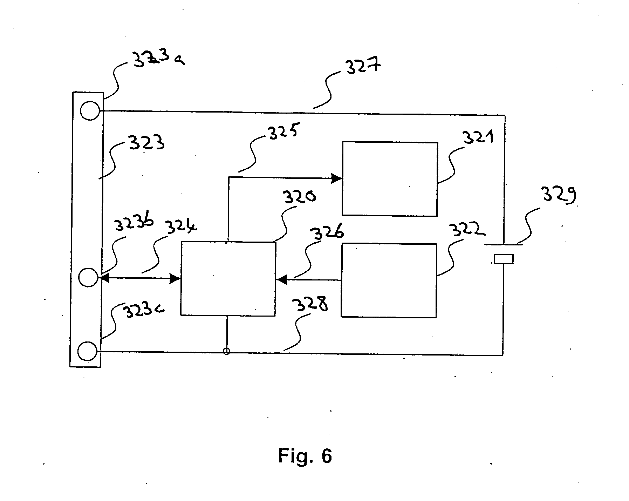

Fig. 6 zeigt ein Blockdiagramm einer Motorsteuerelektronik einer Verbrennungskraftmaschine gemäß einem sechsten Ausführungsbeispiel. Diese Motorsteuerelektronikeinheit weist eine Ionisationsauswerteeinheit 321, eine Zündungsmanagementeinheit 322, ein zweites Kopplungsmittel 320, einen Anschlussstecker 323 sowie eine Spannungsversorgung 329 auf.

Über eine Verbindungsleitung zwischen der Vorrichtung zum Zünden und der Motorelektronik ist der Anschlussstecker 323 der Motorelektronik 327 mit dem Anschlussstecker 307 der Zünd/lonisationseinheit 309 verbunden, die Pole mit gleichen Buchstabenindizes sind elektrisch zusammengeschaltet. Die Spannungsversorgung 329 versorgt über die Leitungen 327 und 328 den Anschlussstecker 323 und damit die Zündeinheit 309 mit Spannung.

Die Zünd/lonisationsleitung 324 der Motorelektronik 327 ist an das Kopplungsmittel 320 der Motorelektronik angeschlossen. Das Kopplungsmittel 320 der Motorelektronik ist ferner über die Verbindungsleitung 326 mit dem Zündmanagement 322 und über die Verbindungsleitung 325 mit der Ionisationsauswertung 321 verbunden.

Zur Zündung leitet das Kopplüngsmittel 320 der Motorelektronik den im Zündmanagement 322 generierten Zündimpuls zum Anschlussstecker Pol 323b und damit zur Zündeinheit 309 weiter. Eine Ionisationsmessspannung von der Zündeinheit, die am Anschlussstecker Pol 323b anliegt wird vom Kopplungsmittel der Motorelektronik 320 über Leitung 325 an die Ionisationsauswertung 321 weitergeleitet.

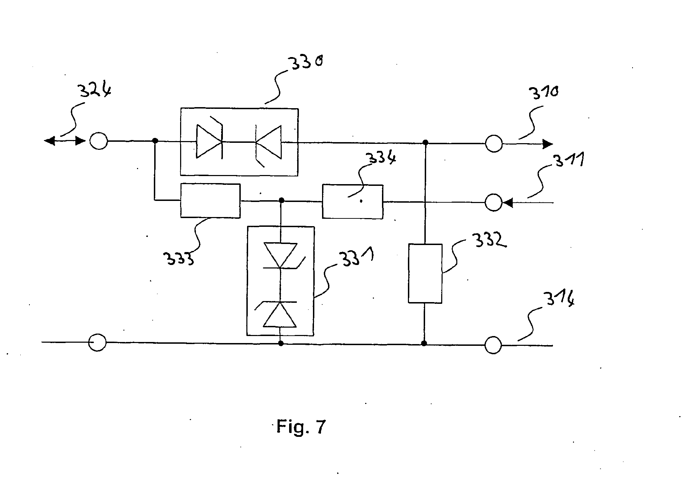

Fig. 7 zeigt ein Blockschaltbild eines zweiten Kopplungsmittels gemäß Fig. 5 gemäß einem siebten Ausführungsbeispiel. Das Kopplungsmittels weist einen ersten spannungsabhängigen Widerstand 330, einen zweiten spannungsabhängigen Widerstand 331, einen ersten Widerstand 332, einen zweiten Widerstand 333 und einen dritten Widerstand 334 auf.

Die eingezeichneten an das Kopplungsmittel angeschlossenen Leitungen korrespondieren mit denen aus Fig. 5. Das Kopplungsmittel 306 selbst enthält einen ersten Pfad zur Weiterleitung des Zündtriggerimpulses bestehend aus dem ersten spannungsabhängigen Widerstand 330 und einem ersten Widerstand 332, der gegen Masse geschaltet ist. Das Kopplungsmittel 306 enthält weiterhin einen zweiten Pfad zur Weiterleitung des Ionisationssignals bestehend aus den zweiten und dritten Widerständen 333 und 334 sowie einen spannungsabhängigen Widerstand 331, der gegen Masse geschaltet ist.

Beide Pfade sind selektiv für die ihnen zugedachten Signalteile leitend, während sie die ihnen nicht zugedachten Signalteile sperren bzw. nicht ungünstig beeinflussen. In Fall einer Zündungsauslösung verhindert Widertand 333 eine unerwünschte Dämpfung des Zündtriggerimpulses, der jedoch die Schwellspannung von spannungsabhängigen Widerstand 330 zur Auslösung einer Zündung passieren kann. Im Fall einer Ionisationsmessung jedoch verhindert die Schwellspannung von spannungsabhängigen Widerstand 330 eine unerwünschte Dämpfung der lonisationsmessspannung, d. h. diese kann die Widerstandskombination 333 und 334 passieren.

Die spannungsabhängigen Widerstände 330 und 331 können als Varistoren, Dioden, Zenerdioden oder einer Kombination dieser Bauteile ausgeführt sein.

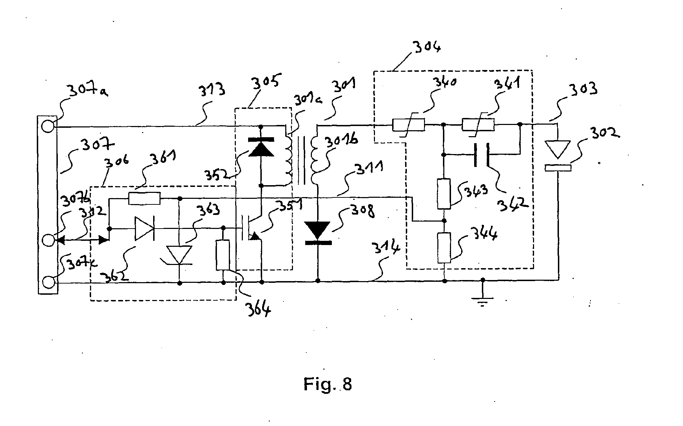

Fig. 8 zeigt ein Schaltbild einer Zündeinheit gemäß einem achten Ausführungsbeispiel. Der Aufbau der Zündeinheit in Fig. 8 entspricht dabei dem Aufbau der Zündeinheit in Fig. 5. Der Ionisationsdetektor 304, das Kopplungsmittel 306 sowie die Leistungselektronik-Zündeinheit 305 sind in Fig. 8 detailliert gezeigt. Das erste Kopplungsmittel 306 weist einen ersten Widerstand 361 und einen zweiten Widerstand 364 auf. Das Kopplungsmittel 306 weist ferner eine erste Diode 362 und eine zweite Diode 363 auf. Die Leistungselektronik-Zündeinheit 305 weist eine Diode 352 und einen Transistor 351 auf. Der Ionisationsdetektor 304 weist einen ersten Widerstand 343 und einen zweiten Widerstand 344, einen Kondensator 342 sowie einer ersten spannungsabhängigen Widerstand 340 und einen zweiten spannungsabhängigen Widerstand 341 auf, wobei die Messspannungs-Speichereinheit durch den Kondensator 342 und den spannungsabhängigen Widerstand 341 (analog zu der Messspannungs-Speichereinheit gemäß Fig. 1, 2 und 3) gebildet wird.

Das Kopplungsmittel beinhaltet die Bauteile 361, 362, 363 und 364. Die Leistungselektronik beinhaltet die Bauelemente 352 und 352. Der Ionisationsdetektor beinhaltet die Bauelemente 340, 341, 342, 343, 344.

Das fünfte, sechste, siebte und achte Ausführungsbeispiel betrifft eine Zündvorrichtung für Verbrennungskraftmaschinen. Die Zündvorrichtung weist eine Zündspule zum Zünden einer Zündkerze, eine Zündeinheit zum Initiieren der Zündung der Zündspule entsprechend einem externen Zündauslösesignal einer externen Motorsteuereinheit, einen Ionisationsdetektor zum Detektieren eines lonisationssignals einer Verbrennung und zum Ausgeben eines dem Ionisationssignal proportionalen lonisationsmesssignals auf. Die Zündvorrichtung weist ferner ein Kopplungsmittel zum selektiven Koppeln des lonisationsmesssignals und des Zündauslösesignals an eine Zünd/lonisationsleitung, welche die Zündvorrichtung mit einer externen Motorsteuereinheit verbindet, auf.

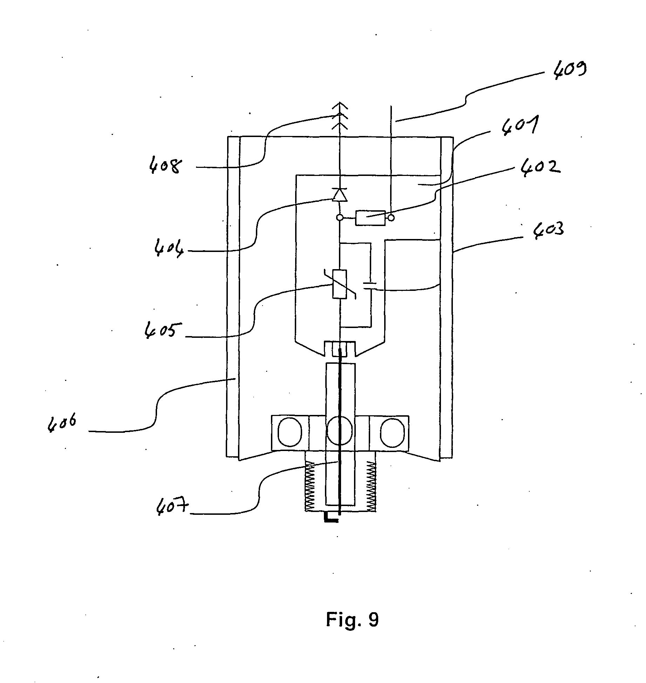

Fig. 9 zeigt eine schematische Darstellung eines Zündkerzensteckers gemäß einem neunten Ausführungsbeispiel. Hier ist insbesondere die Integration einer Ionisationsmessschaltung im Zündkerzenstecker einer Verbrennungskraftmaschine gezeigt.

In dem Zündkerzenstecker 401 sind ein Messwiderstand 402, ein Kondensator 403, ein Entkopplungsbauteil 404 und ein spannungsstabilisierendes Bauteil 405 (Varistor) angeordnet. Der Zündkerzenstecker ist in einem metallischen Steckergehäuse 406 untergebracht, das als Abschirmung gegen elektromagnetische Störungen dient. Der Kondensator 403 und das spannungsstabilisierende Bauteil 405 bilden eine Messspannungs-Speichereinheit, wie sie auch in den vorhergehenden Ausführungsbeispielen beschrieben worden ist. Die in Fig. 9 dargestellte Schaltung mit dem Messwiderstand 402, dem Kondensator 403, dem Entkopplungsbauteil 404 und dem spannungsstabilisierenden Bauteil 405 entspricht im Wesentlichen dem lonisationsmesskreis gemäß Fig. 2 oder gemäß Fig. 8.

Die in Fig. 9 nicht dargestellte Zündspule erzeugt eine Zündspannung in der Größenordnung von beispielsweise10-25 KV. Der Zündstrom passiert über den Anschluss 408 das Entkopplungsbauteil 404, welches als Diode ausgeführt ist in Durchlassrichtung und lädt den parallel geschalteten Messspannungskreis der durch das spannungsstabilisierende Bauteil 405 und dem Kondensator 403 besteht, auf. Nach Beendigung des Funkenüberschlags entkoppelt das Entkopplungsmittel 404 den lonisationsmesskreis von der Zündspule und verhindert den störenden Einfluss der Zündrestspannung auf die Ionisationsmessung. In der dem Funkenabbrand folgenden Verbrennungsphase liegt die aufgeladene Messspannungsspeichereinheit 403,405 über dem Messwiderstand 402 an der Zündelektrode 407 an. Ein dem Ionisationsstrom proportionaler Spannungsabfall ist als Ionisationssignal am Messwiderstand 402 abgreifbar und wird über die Leitung 409 zur weiteren Verarbeitung geleitet.

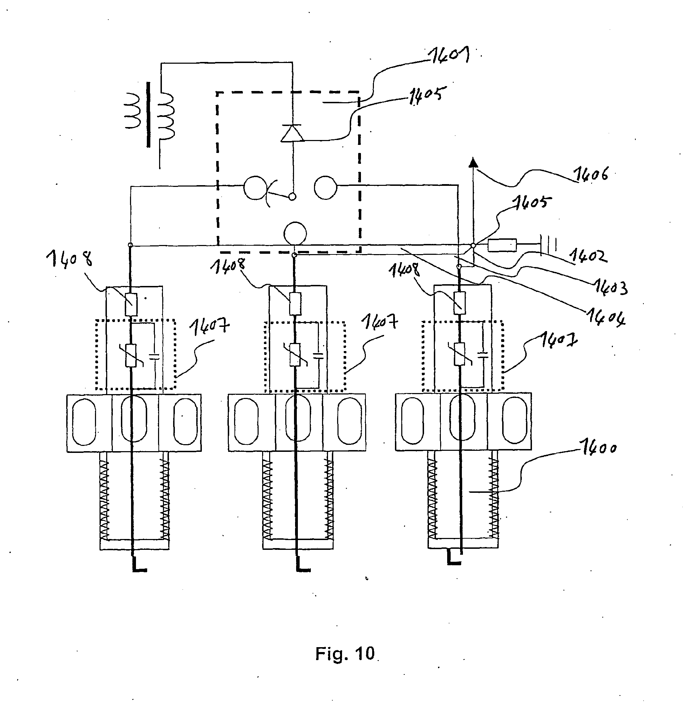

Fig. 10 zeigt eine schematische Darstellung mehrerer Zündkerzen und eines Zündverteilers gemäß einem zehnten Ausführungsbeispiel. Hierbei ist insbesondere die Integrationsmöglichkeit der Messspannungsspeichereinheit 1407, der lonisationsmesseinrichtung 1408 und des Entkopplungsbauteils 1405 innerhalb des Zündverteilers 1401 gezeigt. Die Messspannungs-Speichereinrichtung und die lonisationsmesseinrichtung sowie das Entkopplungsbauteil gemäß einem zehnten Ausführungsbeispiel können der Messspannungs-Speichereinheit, der Ionisationsmesseinrichtung und dem Entkopplungsbauteil gemäß einem der Ausführungsbeispiele eins bis neun entsprechen.

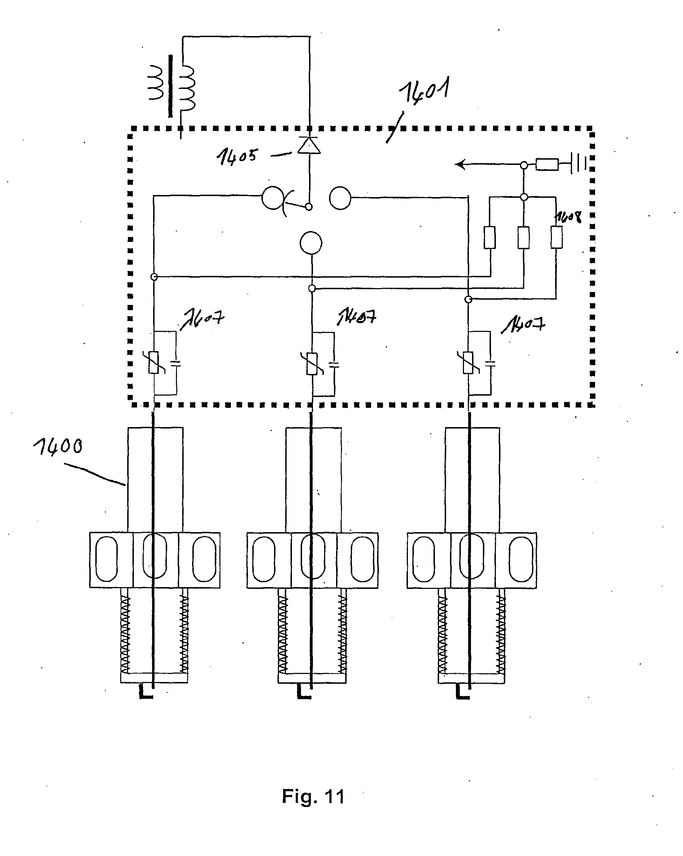

Fig. 11 zeigt eine schematische Darstellung von mehreren Zündkerzen sowie eines Zündverteilers gemäß einem elften Ausführungsbeispiel. Hierbei werden die Messspannungsspeichereinheiten 1407 in den Zündverteiler 1401 integriert. Ein Entkopplungsbauteil 1405 ist mit einem Schalter gekoppelt. Jeder Zündkerze ist eine Messspannungsspeichereinheit 1407 zugeordnet.

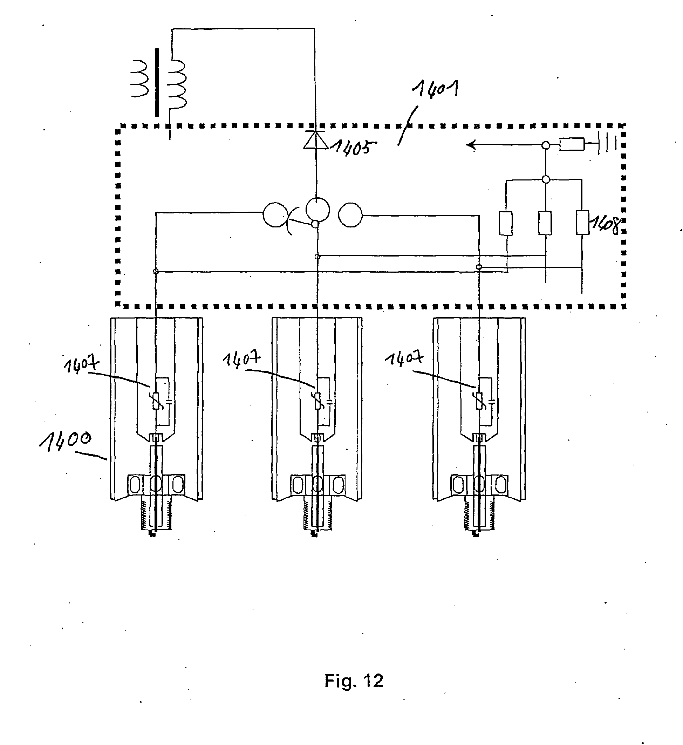

Fig. 12 zeigt eine schematische Darstellung von mehreren Zündkerzen 1407 sowie eines Zündverteilers 1401 einer Verbrennungskraftmaschine gemäß dem zwölften Ausführungsbeispiel. Hierbei ist insbesondere eine Mehrzylindermaschine mit einem Zündverteiler 1401 gezeigt, wobei die Messerfassung 1408 im Zündverteiler 1401 und die Messspannungsquelle 1.407 im Kerzenstecker integriert ist. Hierbei ist insbesondere die Integration der Messspannungsspeichereinheit 1407 innerhalb der Zündkerze gezeigt, während die lonisationsmesseinrichtung und das Entkopplungsbauteil innerhalb des Zündverteilers 401 untergebracht sind.

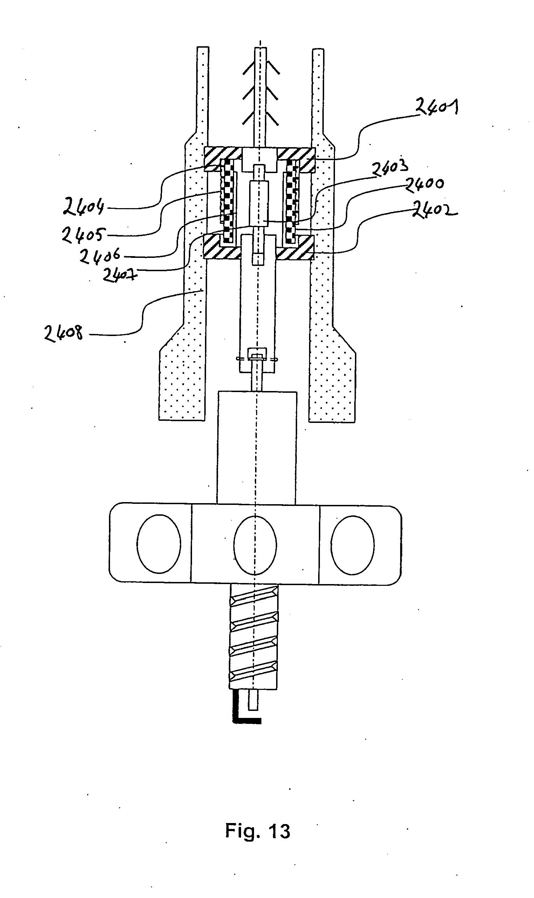

Fig. 13 zeigt eine schematische Darstellung eines Aufbaus eines Zündkerzensteckers gemäß einem dreizehnten Ausführungsbeispiel. Hierbei ist insbesondere eine vorteilhafte Ausgestaltung zur Integration der Messspannungsquelle innerhalb des Zündkerzensteckers oder der Zündkerze gezeigt. Als Energiespeicher wird ein Rohrkondensator 2400 verwendet. Der innerhalb des Rohrkondensators axial angeordnete Varistor 2403 hält die Messspannung während der Verbrennungsphase konstant.

Die Metallische Kontaktierung 2401 bzw. 2402 dient als mechanische Aufnahme der Energiespeichervorrichtung und stellt die Parallelschaltung Rohrkondensator - VDR her.

Die Kondensatorplatten 2405 und 2406 befinden sich am Rohrinnen- und Außenumfang. Die Kondensatorplatten werden mittels der rotationssymmetrischen metallischen Aufnahmen 2401 und 2402 elektrisch verschaltet. Die Kondensatorplatten sind durch eine elektrisch isolierende Schicht 2404 voneinander getrennt. Der Zwischenraum 2407 kann mit keramischer Vergussmasse ausgefüllt sein. Der Außenmantel 2408 ist aus elektrisch isolierenden Material ausgeführt. Der Aufbau kann innerhalb des keramischen Isolators der Zündkerze angeordnet sein.

Obwohl in den oben beschriebenen Ausführungsbeispielen eine Zündspule als Zündspannungserzeugungseinrichtung beschrieben ist, lässt sich die Zündspannungserzeugungseinrichtung auch auf andere Art und Weise realisieren. Dazu wird lediglich eine Vorrichtung benötigt, welche eine Hochspannung entsprechend erzeugen kann. Die Zündspannungserzeugungseinrichtung dient dazu, eine Zündspannung zu erzeugen. Dies kann beispielsweise mittels eines Piezzoaktors erfolgen.

Die in den Fig. 1 bis 3 gezeigte Schaltung kann in einem Zündkerzenstecker, in einer Zündkerze oder in einer Zündverteilungseinrichtung implementiert werden. Die in den Fig. 4, 5, 6, 7 und 8 gezeigte Schaltung kann beispielsweise in einer Zündkerze und/oder in einer Zündverteilungseinrichtung implementiert werden.

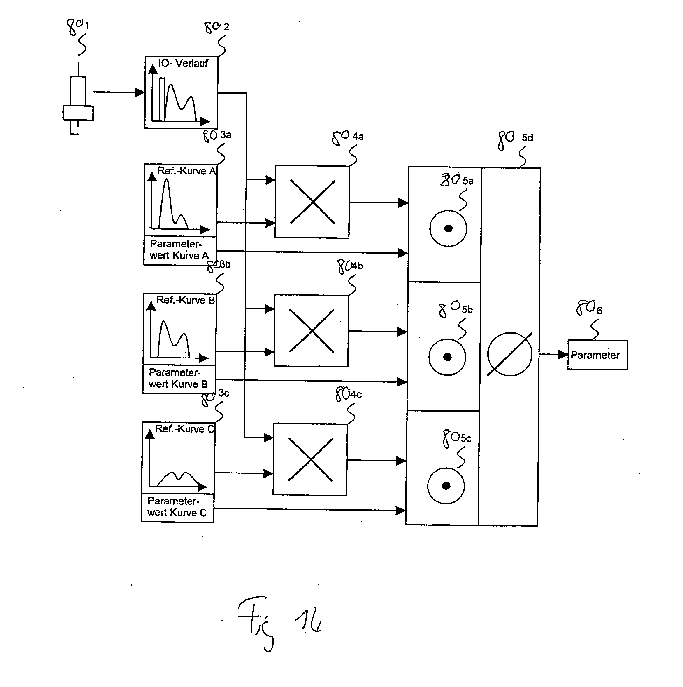

Fig. 14 zeigt eine schematische Darstellung einer Schaltung zum Auswerten von Ionisationssignalverläufen gemäß einem vierzehnten Ausführungsbeispiel. Hierbei kann diese Schaltung zusammen mit den Schaltungen gemäß dem ersten bis zwölften Ausführungsbeispiel verwendet werden. Alternativ dazu kann die Schaltung auch unabhängig davon verwendet werden. In dem Brennraum einer Verbrennungskraftmaschine ist ein Sensor 801 angeordnet. Dieser kann vorteilhafterweise als Zündkerze beim Ottomotor oder als ein elektrisch leitfähiger Stift o.ä. im Brennraum eines Dieselmotors ausgeführt sein.

Das Messsignal wird in einem Messverstärker 802 verstärkt und ggf. von unerwünschten Signalanteilen (z.B. Zündrestspannung) befreit. Weiterhin kann hier eine Mittelung der letzten n Ionisationssignale erfolgen, in diesem Fall wird der gemittelte Verlauf weitergeleitet. Hierbei wird jeweils der Mittelwert aus den Werten gebildet, die den Bezug zur gleichen Referenz haben. Die Referenz kann die verstrichene Zeit nach Zündende oder der Kurbelwinkel oder eine Größe darstellen, welche von dem Kurbelwinkel und der Zeit beeinflusst wird.

Ein Mitteln mehrerer Ionisationssignalverläufe in dem Messverstärker 802 kann auf unterschiedliche Methoden erfolgen. Es werden die letzten n lonisationssignalverläufe komplett gespeichert, bei jedem neu hinzukommenden Verlauf wird der älteste im Speicher befindliche Verlauf gelöscht. Alle im Speicher befindlichen Ionisationswerte werden mit Bezug zum gleichen Punkt auf der x - Achse gemittelt. Somit ergibt sich dann ein gemittelter Verlauf, in dem jeder einzelne Verlauf anteilig gleich stark enthalten ist.

Alternativ dazu kann der aktuelle Ionisationsverlauf mit dem im Speicher befindlichen gemittelten Ionisationsverlauf verrechnet werden, so dass der aktuelle Verlauf den sich im Speicher befindlichen gemittelten Ionisationsverlauf zu einem gewissen Grad aktualisiert. Das Verhältnis des aktuellen Verlaufs der im Speicher befindlichen gemittelten Ionisationsverläufe ergibt die Mittelungstiefe.

Die Mittelungstiefe kann sich an der Dynamik des Motorbetriebspunktes orientieren. Wenn sich der Motor in einem eher stationären Betriebszustand befindet, dann kann die Mittelungstiefe erhöht werden. Wenn sich der Motor jedoch in einem eher instationären Betriebszustand befindet, kann die Mittelungstiefe verringert werden.

In den Referenzsignalspeichereinheiten 803a, 803b, 803c ist jeweils ein einem bestimmten Motorparameterwert zuzuordnender Referenzionisationsverlauf sowie der Wert des Motorparameters gespeichert. Die Anzahl der Referenzsignalspeichereinheiten ist von der gewünschten Auswertegenauigkeit des auszuwertenden Motorparameters sowie dem gewünschten Abstand zur Querempfindlichkeit zu anderen Motorparametern abhängig. Die Anzahl der Referenzsignalspeichereinheiten ist vorzugsweise größer oder gleich zwei.

Wenn die auszuwertende Größe der Motorparameter die Luftzahl darstellt, so ist beispielsweise ein charakteristischer Ionisationssignalverlauf für die Luftzahl Lambda = 0,7 und als Parameterwert die Zahl 0,7 in der Speichereinheit 803a gespeichert. Entsprechend ist in der Speichereinheit (803b)dann ein charakteristischer lonisationssignalverlauf für die Luftzahl Lambda = 1,0 gespeichert, und der Parameterwert entspricht der Zahl 1,0. In der Speichereinheit 803c ist ein charakteristischer Ionisationssignalverlauf für die Luftzahl Lambda = 1,5 gespeichert, und der Parameterwert entspricht der Zahl 1,5.

Die Signalverläufe der Referenzsignalspeichereinheiten 803a - 803c müssen den Bezug zu der gleichen Referenz wie der zu messende lonisationssignalverlauf haben. Die Referenz kann die verstrichene Zeit nach Zündende oder der Kurbelwinkel oder eine von Kurbelwinkel und Zeit beeinflusste Größe darstellen. Die Signalverläufe können entweder als Funktion über die Zeit, als Funktion über den Kurbelwinkel oder als Funktion über eine durch Kurbelwinkel und Zeit beeinflusste Größe aufgetragen sein.

Nachfolgend wird die Generierung der Referenzsignalverläufe beschrieben.

Ein Referenzsignalverlauf wird durch das Betreiben des Motors bei einem Referenzbetriebslauf generiert, bei dem der Parameterwert in Höhe des gewünschten Wertes angefahren wird. Anschließend wird der dazugehörige (gemittelte) lonisationssignalverlauf aufgezeichnet. Ein weiterer Referenzsignalverlauf wird durch das Betreiben des Motors bei einem weiteren Referenzbetriebslauf generiert, bei dem der Parameterwert in Höhe des gewünschten vom ersten Wert abweichenden Wertes angefahren wird und der dazugehörige (gemittelte) lonisationssignalverlauf wird anschließend aufgezeichnet. Die aufgezeichneten parameterwertzugeordneten Ionisationssignalverläufe können dahingehend bearbeitet werden, dass charakteristische Unterschiede in den Signalverläufen verstärkt werden. Weiterhin können die Signalverläufe hinsichtlich der Unterdrückung unerwünschter Parametereinflüsse bearbeitet werden.

Die Korrelationseinheiten 804a, 804b, 804c werden sowohl vom aktuellen lonisationsverlauf (respektive durch einen gemittelten Ionisationsverlauf der letzten n Verläufe) als auch von den jeweils zugeordneten Referenzsignalspeichereinheiten gespeist. Die Korrelationseinheiten 804a - 804c berechnen den Grad der Übereinstimmung zwischen aktuellem Ionisationsverlauf und den jeweils zugeordneten Referenzsignalverlauf. Dies kann erfolgen, indem der Grad der Übereinstimmung jedes Wertes des jeweiligen aktuellen (gemittelten) lonisationssignalverlaufs mit dem zur gleichen Referenz (verstrichene Zeit nach Zündende oder der Kurbelwinkel) gehörigen Wertes des Referenzsignalverlaufes ermittelt wird. Dieser Vergleich wird für alle Werte durchgeführt und der Mittelwert der Grade der Übereinstimmung gebildet.

Nachfolgend wird eine Möglichkeit zur Berechnung des Grades der Übereinstimmung beschrieben.

In einem ersten Schritt erfolgt die punktweise Multiplikation der Verläufe des Ionisationssignals IO und der gespeicherten Referenzverläufe RefA, RefB, wobei die Werte mit Bezug zur gleichen Referenz (verstrichene Zeit nach Zündende oder der Kurbelwinkel) miteinander multipliziert werden. Die Summe aus allen Multiplikationen wird ermittelt. In einem zweiten Schritt erfolgt die punktweise Multiplikation der Referenzkurve mit sich selbst, und eine weitere Summe wird aus diesen Multiplikationen gebildet. In einem dritten Schritt wird die Summe aus den Multiplikationen des Ionisationssignals mit dem Referenzverlauf durch die Summe der Multiplikationen des Referenzverlaufes mit sich selbst geteilt, das Ergebnis stellt den relativen Grad der Übereinstimmung aus der jeweiligen Korrelationseinheit dar.

Die nachfolgende Tabelle 1 zeigt beispielhaft einige Werte der Referenzsignalverläufe RefA, RefB sowie einige Punkte einer gemessenen Ionisationskurve IO. Ferner sind die Zwischenergebnisse des oben beschriebenen Verfahrens gezeigt, d. h. die Multiplikation des Ionisationssignals mit dem ersten Referenzsignal, d. h. IO x RefA, die Multiplikation des Ionisationssignals mit dem zweiten Referenzsignal, d. h. IO x RefB. Ferner ist die Multiplikation des ersten Referenzverlaufs RefA mit sich selbst sowie die Multiplikation des zweiten Referenzsignals RefB mit sich selbst gezeigt.

**Tabelle 1**

| **Punkt** | **Ref A** | **Ref B** | **IO** | **IO*Ref A** | **IO*Ref B** | **Ref A*** | **Ref B*** |
|---|---|---|---|---|---|---|---|
| | | | | | | **Ref A** | **Ref B** |
| 1 | 0 | 0 | 0 | 0 | 0 | 0 | 0 |
| 2 | 0,2 | 0,1 | 0,12 | 0,024 | 0,012 | 0,04 | 0,01 |
| 3 | 0,4 | 0,2 | 0,24 | 0,096 | 0,048 | 0,16 | 0,04 |
| 4 | 0,6 | 0,3 | 0,36 | 0,216 | 0,108 | 0,36 | 0,09 |
| 5 | 0,8 | 0,4 | 0,48 | 0,384 | 0,192 | 0,64 | 0,16 |
| 6 | 1 | 0,5 | 0,6 | 0,6 | 0,3 | 1 | 0,25 |
| 7 | 0,8 | 0,6 | 0,72 | 0,576 | 0,432 | 0,64 | 0,36 |
| 8 | 0,6 | 0,5 | 0,6 | 0,36 | 0,3 | 0,36 | 0,25 |
| 9 | 0,4 | 0,4 | 0,48 | 0,192 | 0,192 | 0,16 | 0,16 |
| 10 | 0,6 | 0,3 | 0,36 | 0,216 | 0,108 | 0,36 | 0.09 |
| 11 | 0,4 | 0,2 | 0,24 | 0,096 | 0,048 | 0,16 | 0,04 |
| 12 | 0,2 | 0,3 | 0,36 | 0,072 | 0,108 | 0,04 | 0,09 |
| 13 | 0 | 0,4 | 0,24 | 0 | 0,096 | 0 | 0,16 |
| 14 | 0 | 0,5 | 0,12 | 0 | 0,06 | 0 | 0,25 |
| 15 | 0 | 0,5 | 0 | 0 | 0 | 0 | 0,25 |
| 16 | 0 | 0,4 | 0 | 0 | 0 | 0 | 0,16 |
| 17 | 0 | 0,3 | 0 | 0 | 0 | 0 | 0,09 |
| 18 | 0 | 0,2 | 0 | 0 | 0 | 0 | 0,04 |
| 19 | 0 | 0,1 | 0 | 0 | 0 | 0 | 0,01 |
| 20 | 0 | 0 | 0 | 0 | 0 | 0 | 0 |
| | | | | | | | |

| | | | | Summe IO*Ref A | Summe IO*Ref B | Summe RefA*RefA | Summe RefB*RefB |
|---|---|---|---|---|---|---|---|
| - | | | | 2,832 | 2,004 | 3,92 | 2,5 |
| | | | | | | | |

| | | | | (Summe IO*Ref A)/ (Ref A*Ref A) | (Summe IO*Ref B)/(Ref B*Ref B) | | |
|---|---|---|---|---|---|---|---|
| | | | | 0,72244898 | 0,8016 | | |
| | | | | | | | |

| | | | | Anteil Ref A | Anteil Ref B | | |
|---|---|---|---|---|---|---|---|
| | | | | 0,474032652 | 0,525967348 | | |

In der Tabelle 1 sind die Referenzsignalverläufe gezeigt, wobei die Zuordnung über die Zeit oder über den Kurbelwinkel erfolgen kann. Ferner sind die der Referenz (Zeit, KW) zugeordneten Werte des Referenzsignalverlaufes A RefA, die der Referenz (Zeit , KW) zugeordneten Werte des Referenzsignalverlaufes B RefB und die der Referenz (Zeit , KW) zugeordneten Werte des lonisationssignalverlaufes IO gezeigt. Darunter sind die jeweiligen Summen der letzten vier Spalten der Tabelle 1 dargestellt. Anschließend ist die Summe aus den Multiplikationen des Ionisationssignals mit dem Referenzverlauf durch die Summe der Multiplikationen des Referenzverlaufes mit sich selbst geteilt dargestellt, und zwar für beide Referenzverläufe. Schließlich werden die Grade der Übereinstimmung zur Summe eins normiert.

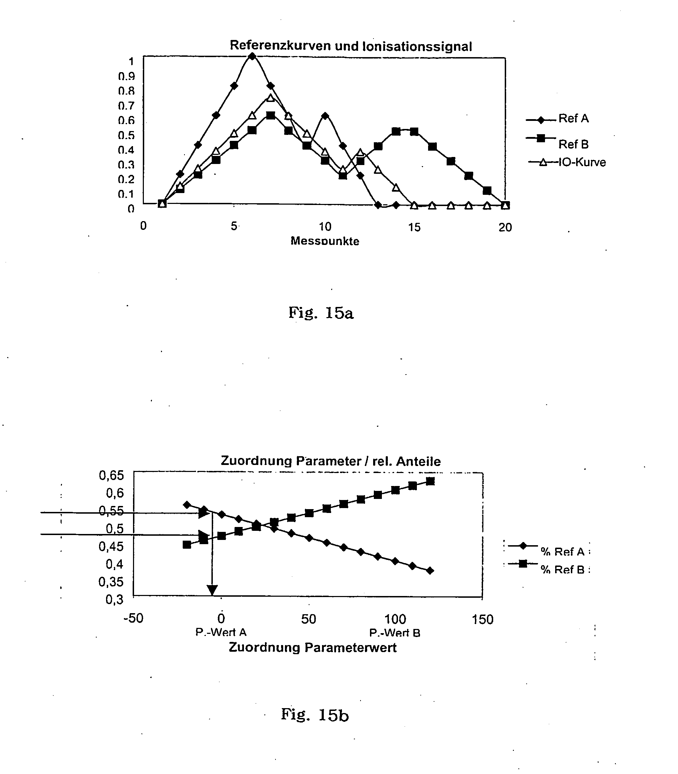

In Fig. 15a ist die relative Signalamplitude der Referenzkurven RefA; RefB sowie der gemessenen Ionisationskurve IO über eine Anzahl von 20 Messpunkten gezeigt. In Fig. 15b sind die relativen Anteile der gemessenen Ionisationskurve IO zu den jeweiligen Referenzsignalverläufen RefA, RefB gezeigt. In dem in der Tabelle 1 gezeigten Fall weist das Ionisationssignal einen Anteil von 47 % an dem Referenzsignalverlauf RefA und einen Anteil von 52 % an dem Referenzsignalverlauf RefB auf.

Der Referenzsignalverlauf RefA entspricht einem Parameterwert von Lambda = 1, und der Referenzsignalverlauf RefB entspricht einem Parameterwert von Lambda = 1,5. Somit ergibt sich ein Lambdawert von 1,24 für die gemessene Ionisationskurve. Der Wert 0 auf der X-Achse von Fig. 15b entspricht einem Lambdawert von Null. Der Wert 100 auf der X-Achse entspricht einem Lambdawert von 1,5. Somit entspricht der ermittelte Wert von ca. 48 einem Lambdawert von 1,24.

Nachfolgend wird eine weitere vorteilhafte Möglichkeit zur Berechnung des Grades der Übereinstimmung beschrieben.

In einem ersten Schritt erfolgt die punktweise Multiplikation der Werte der Verläufe des Ionisationssignals und des Referenzverlaufs mit Bezug zur gleichen Referenz (verstrichene Zeit nach Zündende oder der Kurbelwinkel). Aus dem Ergebnis wird die Quadratwurzel gezogen, und dann wird die Summe aus allen Wurzeln gebildet. In einem zweiten Schritt wird die Summe aller Punkte des Referenzverlaufes gebildet. In einem dritten Schritt wird die Summe aus den Quadratwurzeln der Multiplikationen des Ionisationssignals mit dem Referenzverlauf durch die Summe der Punkte des Referenzverlaufes geteilt, das Ergebnis ist der relative Grad der Übereinstimmung aus der jeweiligen Korrelationseinheit.

Eine weitere Möglichkeit zur Berechnung des Grades der Übereinstimmung ist die punktweise Bewertung der relativen Übereinstimmung (1 bedeutet völlige Übereinstimmung) jedes Wertes des jeweiligen aktuellen (gemittelten) lonisationssignalverlauf mit dem zur gleichen Referenz (verstrichene Zeit nach Zündende oder der Kurbelwinkel) gehörigen Wertes des Referenzsignalverlaufes und die Mittelung dieser Werte.

In den Zuordnungseinheiten 805a, 805b, 805c, 805d wird der von den jeweiligen Korrelationseinheiten 804a, 804b, 804c berechnete Übereinstimmungsgrad den dazugehörigen Parameterwerten aus den Referenzsignalspeichereinheiten 803a, 803b, 803c zugeordnet und in Abhängigkeit der Konstellation der Übereinstimmungsgrade der Parameterwerte berechnet.

Nachfolgend wird eine weitere Möglichkeit zur Berechnung des Parameterwertes des Ionisationssignals beschrieben:

In einem ersten Schritt wird der jeweilige Grad der Übereinstimmung aus den Blöcken 804x mit dem dazugehörigen Parameterwert aus den Referenzsignalspeichereinheiten 803x multipliziert, und anschließend wird die Summe aus den Multiplikationen gebildet. In einem zweiten Schritt wird die Summe der Grade der Übereinstimmungen aus den Blöcken 804x gebildet. In einem dritten Schritt wird die Summe aus den Multiplikationen aus Schritt eins durch die Summe der Übereinstimmungen aus Schritt zwei geteilt. In einem vierten Schritt wird das Ergebnis aus Schritt drei mit Hilfe einer Zuordnungsfunktion dem zu ermittelnden Parameterwert zugeordnet. Die Zuordnungsfunktion wird derart dimensioniert, dass die Beeinflussungen der Zuordnungsqualität durch Überdeckungen der Referenzsignalverläufe kompensiert werden.

Eine weitere vorteilhafte Möglichkeit zur Berechnung des Parameterwertes des Ionisationssignals wird nachfolgend beschrieben:

In einem ersten Schritt werden alle Grade der Übereinstimmung aus den Korrelationseinheiten 804x mit demselben Faktor multipliziert, so dass nach der Multiplikation die Summe der Grade der Übereinstimmungen gleich eins ist. In einem zweiten Schritt werden die normierten Grade der Übereinstimmung aus Schritt eins einer Zuordnungstabelle zugeführt, die für jeweils einen normierten Grad der Übereinstimmung aus der jeweiligen Korrelationseinheit 804x eine Zuordnung zu einem Parameterwert enthält. In einem dritten Schritt wird der Mittelwert aus allen zugeordneten Parameterwerten gebildet und dieser als Parameterwert ausgegeben. Die Zuordnungstabelle kann durch empirische Messungen am Motorprüfstand unter Variation des entsprechenden Parameters oder durch mathematisch - geometrische Rechenoperationen ausgelegt werden.

Gemäß einer weiteren Möglichkeit kann eine n-dimensionale Matrix mit relativen Abhängigkeiten und den dazugehörigen. Parameterwerten erstellt und durch Interpolation den Parameterwert bestimmt werden. Die so ermittelten Parameterwerte können einer weiteren zyklischen Mittelung über n Arbeitstakte der Verbrennungskraftmaschine zugeführt werden. Sollen mehrere verschiedene Motorparameter ausgewertet werden, sind weitere Sätze von Blöcken 803x, 804x und 805x abgestimmt auf die Auswertung des jeweiligen Motorparameters zu implementieren.

Die nachfolgende Tabelle 2 zeigt ein weiteres Beispiel einer weiteren gemessenen Ionisationskurve, wobei die Ionisationskurve gemäß der Tabelle 2 den gleichen Verlauf wie die Ionisationskurve gemäß der Tabelle 1 aufweist, wobei sie lediglich die halbe Amplitude aufweist. Die Verläufe der Referenzsignale RefA und RefB entsprechen den Verläufen aus der Tabelle 1.

**Tabelle 2**

| **Punkt** | **Ref A** | **Ref B** | **IO** | **IO*Ref A** | **IO*Ref B** | **Ref A*** | **Ref B*** |
|---|---|---|---|---|---|---|---|
| | | | | | | **Ref A** | **Ref B** |
| 1 | 0 | 0 | 0 | 0 | 0 | 0 | 0 |
| 2 | 0,2 | 0,1 | 0,06 | 0,012 | 0,006 | 0,04 | 0,01 |
| 3 | 0,4 | 0,2 | 0,12 | 0,048 | 0,024 | 0,16 | 0,04 |
| 4 | 0,6 | 0,3 | 0,18 | 0,108 | 0,054 | 0,36 | 0,09 |
| 5 | 0,8 | 0,4 | 0,24 | 0,192 | 0,096 | 0,64 | 0,16 |
| 6 | 1 | 0,5 | 0,3 | 0,3 | 0,15 | 1 | 0,25 |
| 7 | 0,8 | 0,6 | 0,36 | 0,288 | 0,216 | 0,64 | 0,36 |
| 8 | 0,6 | 0,5 | 0,3 | 0,18 | 0,15 | 0,36 | 0,25 |
| 9 | 0,4 | 0,4 | 0,24 | 0,096 | 0,096 | 0,16 | 0,16 |
| 10 | 0,6 | 0,3 | 0,18 | 0,108 | 0,054 | 0,36 | 0.09 |
| 11 | 0,4 | 0,2 | 0,12 | 0,048 | 0,024 | 0,16 | 0,04 |
| 12 | 0,2 | 0,3 | 0,18 | 0,036 | 0,054 | 0,04 | 0,09 |
| 13 | 0 | 0,4 | 0,12 | 0 | 0,048 | 0 | 0,16 |
| 14 | 0 | 0,5 | 0,06 | 0 | 0,03 | 0 | 0,25 |
| 15 | 0 | 0,5 | 0 | 0 | 0 | 0 | 0,25 |
| 16 | 0 | 0,4 | 0 | 0 | 0 | 0 | 0,16 |
| 17 | 0 | 0,3 | 0 | 0 | 0 | 0 | 0,09 |
| 18 | 0 | 0,2 | 0 | 0 | 0 | 0 | 0,04 |
| 19 | 0 | 0,1 | 0 | 0 | 0 | 0 | 0,01 |
| 20 | 0 | 0 | 0 | 0 | 0 | 0 | 0 |
| | | | | | | | |

| | | | | Summe IO*Ref A | Summe IO*Ref B | Summe RefA*RefA | Summe RefB*RefB |
|---|---|---|---|---|---|---|---|
| | | | | 1,416 | 1,002 | 3,92 | 2,5 |
| | | | | | | | |

| | | | | (Summe IO*Ref A)/ (Ref A*Ref A) | (Summe IO*Ref B)/(Ref B*Ref B) | | |
|---|---|---|---|---|---|---|---|
| | | | | 0,36122449 | 0,4008 | | |
| | | | | | | | |

| | | | | Anteil Ref A | Anteil Ref B | | |
|---|---|---|---|---|---|---|---|
| | | | | 0,474032652 | 0,525967348 | | |

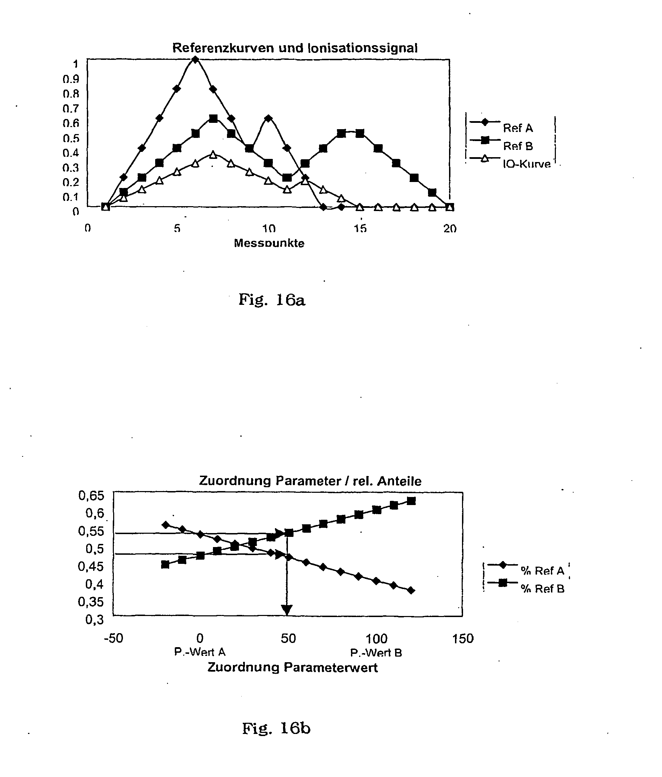

In Fig. 16a und 16b ist ein Graph von Referenzsignalverläufen und einem lonisationssignal sowie ein Graph einer Zuordnung der Parameter gezeigt. Der Verlauf der Referenzkurven RefA und RefB entspricht dem Verlauf dieser Kurven aus Fig. 15a. Aus der Tabelle 2 sowie aus der Fig. 16b ergibt sich, dass die Zuordnung des Lambdawertes (hier 1,24) gemäß der Tabelle 2 sowie den Fig. 16a und 16b der Zuordnung gemäß der Tabelle 1 und den Fig. 15a und 15b entspricht. Als Ergebnis lässt sich somit sagen, dass das oben beschrieben Verfahren zur Ermittlung der jeweiligen Parameter insbesondere von dem Verlauf der lonisationskurve und weniger von der Amplitude der Ionisationskurve abhängt.

Die Tabelle 3 stellt einige Messwerte für die Referenzsignalverläufe RefA, RefB sowie für einen weiteren gemessenen Ionisationssignalverlauf IO dar. Die Referenzsignalverläufe RefA, RefB entsprechen den Referenzsignalverläufen in den Tabellen 1 und 2. Der gemessene Referenzsignalverlauf IO überschreitet teilweise den Bereich der Referenzsignalverläufe RefA, RefB.

**Tabelle 3**

| **Punkt** | **Ref A** | **Ref B** | **IO** | **IO*Ref A** | **IO*Ref B** | **Ref A*** | **Ref B*** |
|---|---|---|---|---|---|---|---|
| | | | | | | **Ref A** | **Ref B** |
| 1 | 0 | 0 | 0 | 0 | 0 | 0 | 0 |
| 2 | 0,2 | 0,1 | 0,075 | 0,015 | 0,0075 | 0,04 | 0,01 |
| 3 | 0,4 | 0,2 | 0,15 | 0,06 | 0,03 | 0,16 | 0,04 |
| 4 | 0,6 | 0,3 | 0,225 | 0,135 | 0,0675 | 0,36 | 0,09 |
| 5 | 0,8 | 0,4 | 0,3 | 0,24 | 0,12 | 0,64 | 0,16 |
| 6 | 1 | 0,5 | 0,375 | 0,375 | 0,1875 | 1 | 0,25 |
| 7 | 0,8 | 0,6 | 0,45 | 0,36 | 0,27 | 0,64 | 0,36 |
| 8 | 0,6 | 0,5 | 0,525 | 0,315 | 0,2625 | 0,36 | 0,25 |
| 9 | 0,4 | 0,4 | 0,6 | 0,24 | 0,24 | 0,16 | 0,16 |
| 10 | 0,6 | 0,3 | 0,525 | 0,315 | 0,1575 | 0,36 | 0.09 |
| 11 | 0,4 | 0,2 | 0,45 | 0,18 | 0,09 | 0,16 | 0,04 |
| 12 | 0,2 | 0,3 | 0,375 | 0,075 | 0,1125 | 0,04 | 0,09 |
| 13 | 0 | 0,4 | 0,3 | 0 | 0,12 | 0 | 0,16 |
| 14 | 0 | 0,5 | 0,225 | 0 | 0,1125 | 0 | 0,25 |
| 15 | 0 | 0,5 | 0,3 | 0 | 0,15 | 0 | 0,25 |
| 16 | 0 | 0,4 | 0,375 | 0 | 0,15 | 0 | 0,16 |
| 17 | 0 | 0,3 | 0,45 | 0 | 0,135 | 0 | 0,09 |
| 18 | 0 | 0,2 | 0,525 | 0 | 0,105 | 0 | 0,04 |
| 19 | 0 | 0,1 | 0,45 | 0 | 0,045 | 0 | 0,01 |
| 20 | 0 | 0 | 0,3 | 0 | 0 | 0 | 0 |
| | | | | | | | |

| | | | | Summe IO*Ref A | Summe IO*Ref B | Summe RefA*RefA | Summe RefB*RefB |
|---|---|---|---|---|---|---|---|
| | | | | 2,31 | 2,3625 | 3,92 | 2,5 |
| | | | | | | | |

| | | | | (Summe IO*Ref A)/ (Ref A*Ref A) | (Summe IO*Ref B)/(Ref B*Ref B) | | |
|---|---|---|---|---|---|---|---|
| | | | | 0,589285714 | 0,945 | | |
| | | | | | | | |

| | | | | Anteil Ref A | Anteil Ref B | | |
|---|---|---|---|---|---|---|---|
| | | | | 0,384078212 | 0,615921788 | | |

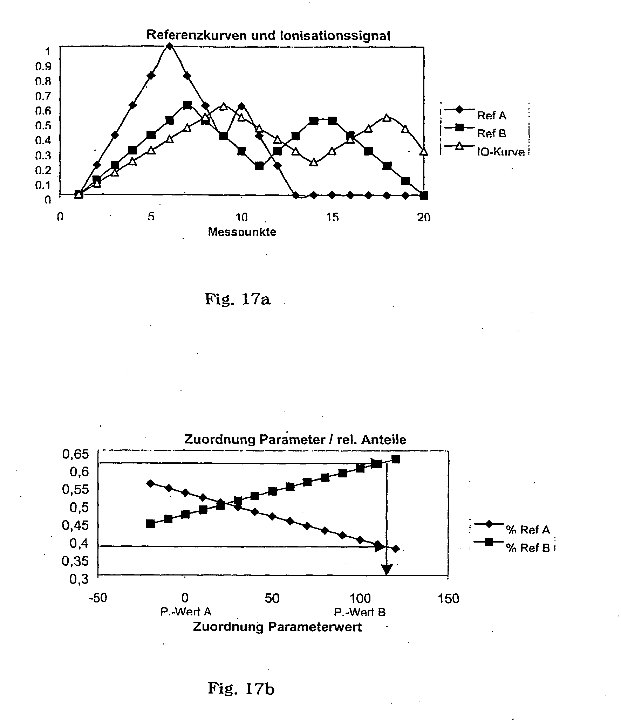

In Fig. 17a ist ein Graph von Referenzkurven sowie eines gemessenen lonisationssignals sowie eine Zuordnung der Parameter gezeigt.

Das oben beschriebene Verfahren hat den Vorteil, dass unabhängig von der absoluten Signalhöhe, welche beispielsweise durch Störparameter wie verschiedene Additive im Brennstoff, Verschmutzung der Elektroden oder dergleichen variieren kann, lediglich die Form des Signalverlaufes entscheidend ist.

Während der gemessen Ionisationsverlauf in der Tabelle 1 und 2 ungefähr zu gleichen Teilen den gespeicherten Referenzsignalverläufen RefA, RefB zugeordnet werden kann, so ist in der Tabelle 3, Fig. 17a, ein gemessener lonisationsverlauf IO gezeigt, welcher sich außerhalb der gespeicherten Referenzsignalverläufe RefA, RefB befindet. Auch ein derartiges Verfahren kann mit dem oben beschriebenen Verfahren ausgewertet werden, obwohl das gemessene Ionisationssignal sich teilweise außerhalb der gespeicherten Referenzsignalverläufe RefA, RefB befindet.

Durch das oben beschriebene Verfahren werden die Punkte eines Referenzsignalverlaufs, welcher sich weiter weg von der gemessenen Ionisationskurve befindet, weniger stark bei der Berechnung berücksichtigt.

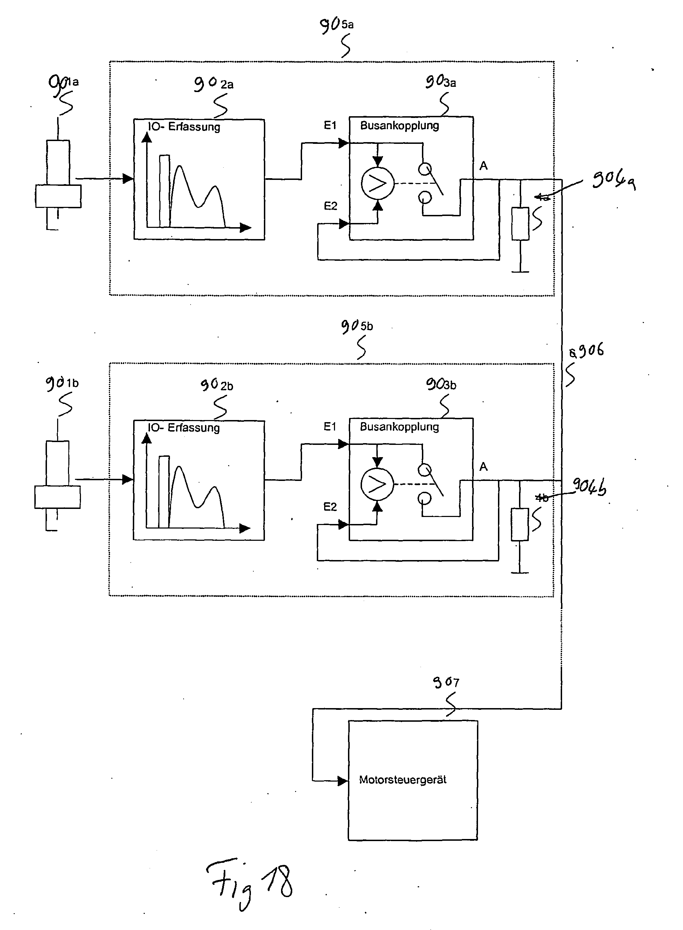

Fig. 18 zeigt ein Blockschaltbild einer Schaltung zum Erfassen von lonisationssignalen verschiedener Zylinder. In jedem Zylinder ist ein Messsensor 901a, 901 b (beispielsweise eine Zündkerze) angeordnet, welche Ionisationssignale erzeugt und an eine Erfassungseinheit 902a, 902b weiterleitet. Die Erfassungseinheiten 902a, 902b sind jeweils mit einer Koppeleinheit 903a, 903b gekoppelt. Die Koppeleinheiten 903a, 903b sind jeweils über eine lonisationssignalübertragungsleitung 906 mit einer Motorsteuerung 907 verbunden. Die Ausgänge der Kopplungseinheiten 903a, 903b sind jeweils über einen Abschlusswiderstand 904a, 904b an die Signalmasse gekoppelt.

Basierend auf der Erkenntnis, dass die Ionisationssignale bei Mehrzylindermaschinen üblicherweise zeitversetzt auftreten, können die Signale von mehreren Zylindern zur Auswertung zusammengefasst und über Koppeleinheiten an eine lonisationssignalübertragungsleitung gekoppelt werden und an eine Motorsteuerung ausgegeben werden.

Das Koppelmittel 903a, 903b schaltet den Ausgang einer Ionisationssignalerfassungseinheit 902a, 902b an die gemeinsame Übertragungsleitung 906, wenn der an dem Ausgang der Erfassungseinheit 902a, 902b anliegende Ionisationswert hinsichtlich seines Betrages größer ist als der an der gemeinsamen Übertragungsleitung 906 anliegende Wert. Somit vergleicht die Koppeleinheit 903a, 903b den Ausgangswert der Erfassungseinheit 902a, 902b mit dem augenblicklichen Wert der gemeinsamen Übertragungsleitung 906.

Alternativ dazu schaltet die Koppeleinheit 903a, 903b den Ausgang der Erfassungseinheit 902a, 902b an die gemeinsame lonisationssignalübertragungsleitung 906 während einer Zeitspanne nach dem Ende der Zündung des jeweiligen Zylinders oder während einer definierten Zeitspanne nach dem Zündende, d. h. während der Zeitdauer, während der ein auswertbares Ionisationssignal vermutet wird.

Vorzugsweise wird die zu einem jeweiligen Zylinder assoziierte Erfassungseinheit 902a, 902b und die Koppeleinheit 903a, 903b jeweils in einem gleichen Gehäuse 905a, 905b (z. B. in einer Stabzündspule) untergebracht. Zusätzlich oder alternativ dazu können alle Koppeleinheiten, welche an eine gemeinsame lonisationssignal-Übertragungsleitung 906 geschaltet sind in einem gemeinsamen Gehäuse untergebracht werden.

## Patentansprüche

1. Schaltung zum Erfassen verbrennungsrelevanter Größen In einer Verbrennungsphase einer Verbrennungskraftmaschine, insbesondere eines Ottomotors, wobei die Verbrennungsphase durch eine mit einem Zündimpuls beaufschlagte Zündelektrode (2) ausgelöst wird, mit
einem Ionisationsmesskreis mit mindestens einem Messwiderstand (9, 10), wobei der Ionisationsmesskreis wenigstens eine Messspannungs-Speichereinheit (6, 7) aufweist, welche mit der Zündelektrode (2) gekoppelt ist und über Entkopplungsmittel (5) von einer Zündspannungserzeugungseinrichtung, insbesondere einer Zündspule (3), der Verbrennungskraftmaschine entkoppelt ist,
**dadurch gekennzeichnet,**
**dass** die Messspannungs-Speichereinheit (6,7) aus einer elektrische Energie speichernden Komponente (7), insbesondere ein Kondensator, und einer spannungsbegrenzenden Komponente (6), insbesondere ein Varistor, eine Zenerdiode, eine Funkenstrecke oder ein Gasableiter, besteht.

2. Schaltung nach Anspruch 1.
**dadurch gekennzeichnet.**
**dass** der Messwiderstand (9, 10) als Reihenschaltung von Widerständen (9, 10) ausgeführt ist.

3. Schaltung nach einem der vorherigen Ansprüche,
**dadurch gekennzeichnet,**
**dass** das Entkopplungsmittel (5,6) aus einer spannungsbegrenzenden Komponente, insbesondere ein Varistor, eine Zenerdiode, eine Funkenstrecke und/oder ein Gasableiter besteht.

4. Schaltung nach einem der Ansprüche 1 bis 3,
**dadurch gekennzeichnet,**
**dass** das Entkopplungsmittel einen der Zündspule (3) parallelgeschalteten Widerstand (8) zur Dämpfung des Ausschwingverhaltens der Zündspule (3) aufweist.

5. Schaltung zur Erfassung verbrennungsrelevanter Größen einer Verbrennungskraftmaschine mit einer lonisationsmesseinrichtung, und mindestens einem Ionisationsmesswiderstand, nach einem der Ansprüche 1 bis 4,
**dadurch gekennzeichnet,**
**dass** ein Messspannungsspeicherkreis (140) zwischen einem ersten Zündspannung führenden Anschluss (101c) der Sekundärseite (101b) des Zündtransformators (101) und der Zündelektrode (102) geschaltet ist, und dass ein elektronischer Schalter (114) zwischen einem Bezugspotential (109) und dem dem Bezugspotential (109) zugeordneten zweiten Anschluss (101d) der Sekundärseite (101b) des Zündtransformators (101) geschaltet ist.

6. Schaltung nach Anspruch 5,
**dadurch gekennzeichnet,**
**dass** eine weitere Entkoppeleinrichtung (105) zwischen dem die Zündspannung führenden Anschluss (101 c) der Sekundärseite (101 b) des Zündtransformators (101) und der Ionisationsmessschaltung (130) geschaltet ist.

7. Schaltung nach Anspruch 6,
**dadurch gekennzeichnet,**
**dass** die Entkoppeleinrichtung (105) eine Diode, eine Zenerdiode und/oder einen Varistor aufweist.

8. Schaltung nach einem der Ansprüche 5 bis 7,
**dadurch gekennzeichnet,**
**dass** der Messspannungsspeicherkreis (140) aus einer Parallelschaltung eines spannungsstabilisierenden Bauelementes (103) und einem spannungsspeichernden Beuelement (104) besteht.

9. Schaltung nach einem der Ansprüche 5 bis 8,
**dadurch gekennzeichnet,**
**dass** der elektronische Schalter (114) über Koppelbauelemente (123) an den Strompfad (121) des Primärkreises des Zündtransformators (101) angekoppelt ist.

10. Schaltung nach einem der Ansprüche 5 bis 9,
**dadurch gekennzeichnet,**
**dass** zwischen dem dem Bezugspotential (109) zugeordneten Anschluss (101d) der Sekundärseite (101b) des Zündtransformators (101) und dem Ionisationsmessausgang (108) ein Koppelelement (124) zur Kompensation geschaltet ist.

11. Schaltung nach Anspruch 10,
**dadurch gekennzeichnet,**
**dass** das Koppelelement (124) ein Widerstand, eine Widerstands/Kondensator-Kombination oder eine Verstärkerstufe ist.

12. Verfahren zur Erfassung verbrennungsrelevanter Größen eines Verbrennungsprozesses durch eine Schaltung nach einem der Ansprüche 1 bis 11 in einer Verbrennungsphase einer Verbrennungskraftmaschine, mit den Schritten
Initiieren einzelner Verbrennungsvorgänge durch einen Zündimpuls.
Ermitteln der verbrennungsrelevanten Größen in Abhängigkeit eines durch die Flamme beeinflussten Ionisationssignals,
Aufbauen eines Spannungspotentials in einer Messspannungs-Speichereinheit (6, 7) in der Zündphase durch einen Zündstrom.
Anliegen eines Spannungspotenzials in wenigstens einer Messphase über einem Messwiderstand (10) an einer Zündelektrode (2), wobei die Zündspannung bei Unterschreiten einer Schwellspannung die Messspannungs-Speichereinheit (6, 7) oder den Messwiderstand (9, 10) von der Messspannung entkoppelt wird,
wobei die dem Ionisationssignal proportionale Spannung an einem der Widerstände abgegriffen wird und an eine Auswerteeinheit (11) weitergeleitet wird.

13. Verfahren zum Erfassen verbrennungsrelevanter Größen in der Verbrennungsphase einer Verbrennungskraftmaschine mittels einer mit einem Zündimpuls beaufschlagten Zündelektrode, nach Anspruch 12, mit einem Ionisationsmesskreis und zur Ionisationsmessung mit einer wenigstens im Anschluss an die Zündung an die Zündelektrode angelegten Messspannung,
**dadurch gekennzeichnet,**
**dass** der Ionisationsmesskreis (130) zwischen dem Zündspannung führenden Anschluss (101c) der Sekundärseite (101b) des Zündtransformators (101) und der Zündelektrode (102) geschaltet ist und
**dass** zwischen dem Bezugspotential (109) und ein dem Bezugspotential (109) zugeordneten Anschluss (101d) der Sekundärseite (101b) des Zündtransformators (101) ein elektronischer Schalter (114) geschaltet ist, der durch eine Steuereinheit (112) während des Zündimpulses zur Weiterleitung des Zündsignals leitend geschaltet wird und zumindest während der Ionisationsmessphase sperrend geschaltet wird.

14. Verfahren nach Anspruch 13,
enthaltend den Verfahrensschritt.
dass die Steuereinheit (112) den elektronischen Schalter (114) zumindest während der Dauer der Abschaltung des Zündimpulses sperrend schaltet, wobei die Steuerelektronik mit Mitteln (120, 122) zur Abschaltung des Zündimpulses durch niederimpedante Belastung der Primärwicklung (101a) des Zündtransformators (101) ausgestattet ist.

15. Verfahren nach Anspruch 14,
enthaltend den Verfahrensschritt,
dass die Steuereinheit (112) den elektronischen Schalter (114) schon kurz vor der Abschaltung des Zündimpulses sperrend schaltet.

16. Zündtransformator mit einer Schaltung zur Erfassung verbrennungsrelevanter Größen einer Verbrennungskraftmaschine mit einer Ionisationsmesselnrichtung, und mindestens einem Ionisationsmesswiderstand, nach einem der Ansprüche 1 bis 11,
**dadurch gekennzeichnet,**
**dass** der Messspannungsspeicherkreis (140) zwischen dem Zündspannung führenden Anschluss (101c) der Sekundärseite (101b) des Zündtransformators (101) und der Zündelektrode (102) geschaltet ist, und dass ein elektronischer Schalter (114) zwischen einem Bezugspotential (109) und dem dem Bezugspotential (109) zugeordneten Anschluss (101d) der Sekundärseite (101b) des Zündtransformators (101) geschaltet ist,

17. Zündvorrichtung für Verbrennungskraftmaschinen, mit
einer Zündspule (301) zum Zünden einer Zündkerze (302) der Verbrennungskraftmaschine,
einer Zündeinheit (305) zum Initiieren der Zündung der Zündspule (301) entsprechend einem externen Zündauslösesignal von einer externen Motorsteuereinheit,
einem Ionisationsdetektor (304) zum Delektieren eines Ionisationssignals einer Verbrennung der Verbrennungskraftmaschine und zum Ausgeben eines zu dem Ionisationssignal proportionalen Ionisationsmesssignals, und
einem Kopplungsmittel (306) zum selektiven Koppeln des Ionisationsmesssignals und des Zündauslösesignals an eine Zünd-/Ionisationsleitung (312), welche die Zündvorrichtung mit der externen Motorsteuereinheit verbindet,
wobei das Kopplungsmittel (306) einen ersten Pfad zur Weiterleitung des Zündauslösesignals und einen zweiten Pfad zur Weiterleitung des Ionisationssignals aufweist, wobei der erste Pfad für das Zündauslösesignal leitend und für das Ionisationsmesssignal sperrend ausgestaltet ist, wobei der zweite Pfad für das Ionisationsmesssignal leitend und für das Zündauslösesignel sperrend ausgestaltet ist.
wobei der Ionisationsdetektor eine Schaltung zum Erfassen verbrennungsrelevanter Größen in einer Verbrennungsphase einer Verbrennungskraftmaschine nach einem der Ansprüche 1 bis 11 aufweist.

18. Zündkerzenstecker mit einer Schaltung zum Erfassen verbrennungsrelevanter Größen gemäß einem der Ansprüche 1 bis 11.

19. Zündkerzenvorrichtung mit einer Schaltung zum Erfassen verbrennungsrelevanter Größen gemäß einem der Ansprüche 1 bis 11,

20. Motorzündvortichtung zum Zünden von Verbrennungen in einer Verbrennungskraftmaschine, mit
einer Zündkerze nach Anspruch 19, und
einer Zündverteilungseinrichtung (401), welche ein Entkopplungsbauteil (404) aufweist.

21. Motorzündvorrichtung zum Zünden von Verbrennungen in einer Verbrennungskraftmaschine, mit
einer Vielzahl von Zündkerzen, und
einer Zündverteilungseinrlchtung (1401), wobei die Zündverteilungseinrichtung wenigstens eine Schaltung zum Erfassen verbrennungsrelevanter Größen gemäß einem der Ansprüche 1 bis 11 aufweist,
wobei die Zündverteilungselnrichtung (1401) ein Entkopplungsbauteil (1405) aufweist, und wobei Messwiderstände (1408) mit einem gemeinsamen Stempunkt gekoppelt sind.

## Claims

1. Circuit for detecting combustion-relevant variables in a combustion phase of an internal combustion engine, in particular a spark ignition engine, wherein the combustion phase is triggered by an ignition electrode (2) supplied with an ignition pulse, comprising
an ionisation measurement circuit having at least one measurement resistor (9, 10),
wherein the ionisation measurement circuit has at least one measurement voltage storage unit (6, 7) which is coupled to the ignition electrode (2) and is decoupled by way of decoupling means (5) from an ignition voltage generating device, in particular an ignition coil (3), of the internal combustion engine,
**characterised**
**in that** the measurement voltage storage unit (6, 7) comprises an electrical energy-storing component (7), in particular a capacitor, and a voltage-limiting component (6), in particular a varistor, a Zener diode, a spark gap or a gas arrester.

2. Circuit according to Claim 1,
**characterised**
**in that** the measurement resistor (9, 10) is in the form of a series connection of resistors (9, 10).

3. Circuit according to one of the preceding claims,
**characterised**
**in that** the decoupling means (5, 6) comprises a voltage-limiting component, in particular a varistor, a Zener diode, a spark gap and/or a gas arrester.

4. Circuit according to one of Claims 1 to 3,
**characterised**
**in that** the decoupling means has a resistor (8) connected in parallel with the ignition coil (3) for damping the transient response of the ignition coil (3).

5. Circuit for detecting combustion-relevant variables of an internal combustion engine comprising an ionisation measurement device, and at least one ionisation measurement resistor, according to one of Claims 1 to 4,
**characterised**
**in that** a measurement voltage storage circuit (140) is connected between a first ignition voltage-carrying connection (101c) of the secondary side (101b) of the ignition transformer (101) and the ignition electrode (102), and in that an electronic switch (114) is connected between a reference potential (109) and the second connection (101d), associated with the reference potential (109), of the secondary side (101b) of the ignition transformer (101).

6. Circuit according to Claim 5,
**characterised**
**in that** a further decoupling device (105) is connected between the ignition voltage-carrying connection (101c) of the secondary side (101b) of the ignition transformer (101) and the ionisation measurement circuit (130).

7. Circuit according to Claim 6,
**characterised**
**in that** the decoupling device (105) has a diode, a Zener diode and/or a varistor.

8. Circuit according to one of Claims 5 to 7,
**characterised**
**in that** the measurement voltage storage circuit (140) comprises a parallel circuit of a voltage-stabilizing component (103) and a voltage-storing component (104).

9. Circuit according to one of Claims 5 to 8,
**characterised**
**in that** the electronic switch (114) is coupled by way of coupling components (123) to the current path (121) of the primary circuit of the ignition transformer (101).

10. Circuit according to one of Claims 5 to 9,
**characterised**
**in that** a coupling element (124) for compensation purposes is connected between the connection (101d), associated with the reference potential (109), of the secondary side (101b) of the ignition transformer (101) and the ionisation measurement output (108).

11. Circuit according to Claim 10,
**characterised**
**in that** the coupling element (124) is a resistor, a resistor/capacitor combination or an amplifier stage.

12. Method for detecting combustion-relevant variables of a combustion process by a circuit according to one of Claims 1 to 11 in a combustion phase of an internal combustion engine, comprising the steps of
initiating individual combustion processes by an ignition pulse,
ascertaining the combustion-relevant variables in dependence on an ionisation signal influenced by the flame,
building up a voltage potential in a measurement voltage storage unit (6, 7) in the ignition phase by an ignition current,
applying a voltage potential in at least one measurement phase by way of a measurement resistor (10) to an ignition electrode (2), wherein the ignition voltage, when it falls below a threshold voltage, is decoupled the measurement voltage storage unit (6, 7) or the measurement resistor (9, 10) from the measurement voltage,
wherein the voltage which is proportional to the ionisation signal is detected at one of the resistors and passed to an evaluation unit (11).

13. Method for detecting combustion-relevant variables in the combustion phase of an internal combustion engine by means of an ignition electrode supplied with an ignition pulse, according to Claim 12, with an ionisation measurement circuit, and for ionisation measurement with a measurement voltage applied to the ignition electrode at least subsequently to the ignition,
**characterised**
**in that** the ionisation measurement circuit (130) is connected between the ignition voltage-carrying connection (101c) of the secondary side (101b) of the ignition transformer (101) and the ignition electrode (102), and
**in that** an electronic switch (114) is connected between the reference potential (109) and a connection (101d), associated with the reference potential (109), of the secondary side (101b) of the ignition transformer (101), which electronic switch (114) is switched into the conducting condition by a control unit (112) during the ignition pulse for passing the ignition signal and is switched into the non-conducting condition at least during the ionisation measurement phase.

14. Method according to Claim 13,
including the method step
that the control unit (112) switches the electronic switch (114) into the non-conducting condition at least during the duration of switching off the ignition pulse, wherein the control electronics are provided with means (120, 122) for switching off the ignition pulse by low-impedance loading of the primary winding (101a) of the ignition transformer (101).

15. Method according to Claim 14,
including the method step
that the control unit (112) already switches the electronic switch (114) into the non-conducting condition shortly before the ignition pulse is switched off.

16. Ignition transformer comprising a circuit for detecting combustion-relevant variables of an internal combustion engine comprising an ionisation measurement device, and at least one ionisation measurement resistor, according to one of Claims 1 to 11,
**characterised**
**in that** the measurement voltage storage circuit (140) is connected between the ignition voltage-carrying connection (101c) of the secondary side (101b) of the ignition transformer (101) and the ignition electrode (102), and in that an electronic switch (114) is connected between a reference potential (109) and the connection (101d), associated with the reference potential (109), of the secondary side (101b) of the ignition transformer (101).

17. Ignition device for internal combustion engines, comprising
an ignition coil (301) for ignition of a spark plug (302) of the internal combustion engine,
an ignition unit (305) for initiating ignition of the ignition coil (301) in accordance with an external ignition triggering signal from an external engine control unit,
an ionisation detector (304) for detecting an ionisation signal of combustion of the internal combustion engine and for outputting an ionisation measurement signal which is proportional to the ionisation signal, and
a coupling means (306) for selectively coupling the ionisation measurement signal and the ignition triggering signal to an ignition/ionisation line (312) which connects the ignition device to the external engine control unit,
wherein the coupling means (306) has a first path for passing the ignition triggering signal and a second path for passing the ionisation signal, wherein the first path is adapted to be conducting for the ignition triggering signal and non-conducting for the ionisation measurement signal, wherein the second path is adapted to be conducting for the ionisation measurement signal and non-conducting for the ignition triggering signal,
wherein the ionisation detector has a circuit for detecting combustion-relevant variables in a combustion phase of an internal combustion engine according to one of Claims 1 to 11.

18. Spark plug connector comprising a circuit for detecting combustion-relevant variables according to one of Claims 1 to 11.

19. Spark plug device comprising a circuit for detecting combustion-relevant variables according to one of Claims 1 to 11.

20. Engine ignition device for the ignition of combustion processes in an internal combustion engine, comprising
a spark plug according to Claim 19, and
an ignition distribution device (401) having a decoupling component (404).

21. Engine ignition device for the ignition of combustion processes in an internal combustion engine, comprising
a multiplicity of spark plugs, and
an ignition distribution device (1401), wherein the ignition distribution device has at least one circuit for detecting combustion-relevant variables according to one of Claims 1 to 11,
wherein the ignition distribution device (1401) has a decoupling component (1405), and wherein measurement resistors (1408) are coupled with a common star point.

## Revendications

1. Circuit permettant de saisir des grandeurs concernant la combustion lors d'une phase de combustion d'une machine à combustion interne, en particulier d'un moteur à essence, la phase de combustion étant déclenchée par une électrode (2) d'allumage alimentée par une impulsion d'allumage, avec
un circuit de mesure d'ionisation avec au moins une résistance (9,10) de mesure, le circuit de mesure d'ionisation présentant au moins une unité (6,7) mémoire de tension de mesure couplée à l'électrode (2) d'allumage et désaccouplée d'un dispositif de production de tension d'allumage de la machine à combustion interne, en particulier une bobine (3) d'allumage par un élément (5) de désaccouplement ,
**caractérisé en ce que**
l'unité (6,7) mémoire de tension de mesure est constituée d'un composant (7) accumulant l'énergie électrique, en particulier un condensateur, et d'un composant (6) limitant la tension, en particulier une varistance, une diode Zener, un éclateur ou un éclateur à gaz.

2. Circuit selon la revendication 1,
**caractérisé en ce que**
la résistance (9,10) de mesure est réalisée comme un circuit de résistances (9,10) en série.

3. Circuit selon l'une des revendications précédentes,
**caractérisé en ce que**
l'élément (5,6) de désaccouplement est constitué d'un composant limitant la tension, en particulier une varistance, une diode Zener, un éclateur et/ou un éclateur à gaz.

4. Circuit selon l'une des revendications 1 à 3,
**caractérisé en ce que**
l'élément de désaccouplement présente une résistance (8) connectée en parallèle à la bobine (3) d'allumage afin d'atténuer le comportement d'extinction d'oscillation de la bobine (3) d'allumage.

5. Circuit permettant de saisir des grandeurs concernant la combustion d'une machine à combustion interne avec un dispositif de mesure d'ionisation et au moins une résistance de mesure d'ionisation selon l'une des revendications 1 à 4,
**caractérisé en ce**
**qu'**un circuit mémoire (140) de tension de mesure est connecté entre un premier raccordement (101c) du côté secondaire (101b) du transformateur (101) d'allumage, conduisant la tension d'allumage, et l'électrode (102) d'allumage, et en ce qu'un commutateur électronique (114) est connecté entre un potentiel (109) de référence et le deuxième raccordement (101d) du côté secondaire (101b) du transformateur (101) d'allumage, affecté au potentiel (109) de référence.

6. Circuit selon la revendication 5,
**caractérisé en ce**
**qu'**un autre dispositif (105) de désaccouplement est connecté entre le raccordement du côté secondaire (101b) du transformateur (101) d'allumage, conduisant la tension d'allumage, et le circuit (130) de mesure d'ionisation.

7. Circuit selon la revendication 6,
**caractérisé en ce que**
le dispositif (105) de désaccouplement présente une diode, une diode Zener et/ou une varistance.

8. Circuit selon l'une des revendications 5 à 7,
**caractérisé en ce que**
le circuit mémoire (140) de tension de mesure est constitué d'un circuit en parallèle constitué d'un composant (103) stabilisant la tension et d'un composant (104) accumulant la tension.

9. Circuit selon l'une des revendications 5 à 8,
**caractérisé en ce que**
le commutateur électronique (114) est couplé au trajet (121) de courant du circuit primaire du transformateur (101) d'allumage via des composants (123) d'accouplement.

10. Circuit selon l'une des revendications 5 à 9,
**caractérisé en ce**
**qu'**un élément (124) d'accouplement destiné à la compensation est connecté entre le raccordement (101d) du côté secondaire (101b) du transformateur (101) d'allumage, affecté au potentiel (109) de référence et la sortie (108) de mesure d'ionisation.

11. Circuit selon la revendication 10,
**caractérisé en ce que**
l'élément (124) d'accouplement est une résistance, une combinaison résistance/condensateur ou un étage amplificateur.

12. Procédé de saisie de grandeurs concernant la combustion d'un processus de combustion par un circuit selon l'une des revendications 1 à 11 lors d'une phase de combustion d'une machine à combustion interne, comprenant les étapes
initier différents processus de combustion par une impulsion d'allumage, calculer les grandeurs concernant la combustion en fonction d'un signal d'ionisation influencé par la flamme,
réaliser un potentiel de tension dans une unité mémoire (6,7) de tension de mesure par un courant d'allumage lors de la phase d'allumage, appliquer un potentiel de tension à une électrode (2) d'allumage via une résistance (10) de mesure lors d'au moins une phase de mesure, la tension d'allumage lors du dépassement bas d'une tension seuil de l'unité mémoire (6,7) de tension de mesure ou la résistance (9,10) de mesure étant désaccouplée de la tension de mesure, la tension proportionnelle au signal d'ionisation étant mesurée sur l'une des résistances et étant transmise à une unité (11) d'analyse.

13. Procédé de saisie de grandeurs concernant la combustion lors de la phase de combustion d'une machine à combustion interne au moyen d'une électrode d'allumage alimentée par une impulsion d'allumage selon la revendication 12, avec un circuit de mesure d'ionisation et permettant de mesurer l'ionisation par une tension de mesure appliquée à l'électrode d'allumage au moins après l'allumage,
**caractérisé en ce que**
le circuit (130) de mesure d'ionisation est connecté entre le raccordement (101c) du côté secondaire (101b) du transformateur (101) d'allumage, conduisant la tension d'allumage, et l'électrode (102) d'allumage,
et **en ce qu'**un commutateur électronique (114) est connecté entre le potentiel (109) de référence et un connecteur (101d) du côté secondaire (101b) du transformateur (101) d'allumage, affecté au potentiel (109) de référence, commutateur connecté de façon conductrice par une unité (112) de commande pendant l'impulsion d'allumage afin de transmettre le signal d'allumage et qui est mis à l'état bloqué au moins pendant la phase de mesure d'ionisation.

14. Procédé selon la revendication 13,
comprenant l'étape de procédé
que l'unité (112) de commande met le commutateur électronique (114) à l'état bloqué au moins pendant la durée de la désactivation de l'impulsion d'allumage, l'électronique de commande étant dotée d'éléments (120, 122) de désactivation de l'impulsion d'allumage par une charge à faible impédance de l'enroulement primaire (101a) du transformateur (101) d'allumage.

15. Procédé selon la revendication 14,
comprenant l'étape de procédé
que l'unité (112) de commande met déjà le commutateur électronique (114) à l'état bloqué légèrement avant la désactivation de l'impulsion d'allumage.

16. Transformateur d'allumage avec un circuit permettant de saisir des grandeurs concernant la combustion d'une machine à combustion interne avec un dispositif de mesure d'ionisation et au moins une résistance de mesure d'ionisation, selon l'une des revendications 1 à 11,
**caractérisé en ce que**
le circuit mémoire (140) de tension de mesure est connecté entre le raccordement (101c) du côté secondaire (101b) du transformateur (101) d'allumage, conduisant la tension d'allumage, et l'électrode (102) d'allumage, et **en ce qu'**un commutateur électronique (114) est connecté entre un potentiel (109) de référence et le raccordement (101d) du côté secondaire (101b) du transformateur (101) d'allumage, affecté au potentiel (109) de référence.

17. Dispositif d'allumage pour machines à combustion interne, avec une bobine (301) d'allumage permettant d'allumer une bougie (302) d'allumage de la machine à combustion interne
une unité (305) d'allumage permettant d'initier l'allumage de la bobine (301) d'allumage en fonction d'un signal externe de déclenchement d'allumage d'une unité de commande de moteur,
un détecteur (304) d'ionisation permettant de détecter un signal d'ionisation d'une combustion de la machine à combustion interne et d'émettre un signal de mesure d'ionisation proportionnel au signal d'ionisation, et
un élément (306) d'accouplement permettant de coupler sélectivement le signal de mesure d'ionisation et le signal de déclenchement d'allumage à un câble (312) d'allumage/d'ionisation, lequel relie le dispositif d'allumage à l'unité de commande de moteur externe,
l'élément (306) d'accouplement présentant un premier trajet, pour la transmission du signal de déclenchement d'allumage, et un deuxième trajet, pour la transmission du signal d'ionisation, le premier trajet étant conçu pour être conducteur du signal de déclenchement d'allumage et pour être à l'état bloqué pour le signal de mesure d'ionisation, le deuxième trajet étant conçu pour être conducteur du signal de mesure d'ionisation et pour être à l'état bloqué pour le signal de déclenchement d'allumage,
le détecteur d'ionisation présentant un circuit permettant de saisir des grandeurs concernant la combustion lors d'une phase de combustion d'une machine à combustion interne selon l'une des revendications 1 à 11.

18. Connecteur pour bougie d'allumage avec un circuit permettant de saisir des grandeurs concernant la combustion selon l'une des revendications 1 à 11.

19. Dispositif de bougies d'allumage avec un circuit permettant de saisir des grandeurs concernant la combustion selon l'une des revendications 1 à 11.

20. Dispositif d'allumage de moteur pour l'amorçage de combustions dans une machine à combustion interne, avec
une bougie d'allumage selon la revendication 19 et
un dispositif (401) de distribution d'allumage qui présente un composant (404) de désaccouplement.

21. Dispositif d'allumage de moteur pour l'amorçage de combustions dans une machine à combustion interne, avec
une multitude de bougies d'allumage et
un dispositif (1401) de distribution d'allumage, le dispositif de distribution d'allumage présentant au moins un circuit permettant de saisir des grandeurs concernant la combustion selon l'une des revendications 1 à 11,
le dispositif (1401) de distribution d'allumage présentant un composant (1405) de désaccouplement et les résistances (1408) de mesure étant couplées à un point neutre commun.
